# EUROPÄISCHE PATENTANMELDUNG

(11) **EP 1 531 129 A1**
(43) Veröffentlichungstag der Anmeldung: **18.05.2005**
(21) Anmeldenummer: 04027184.3
(22) Anmeldetag: 16.11.2004
(51) Int. Cl.: B65D 47/00, B05B 11/00, A61K 7/00, A61K 9/10

(54) **Kosmetikum mit empfindlichen Inhaltsstoffen**

(30) Priorität: 17.11.2003 DE 10354052
(71) Anmelder: Beiersdorf AG, 20253 Hamburg (DE)
(72) Erfinder: Lanzendörfer, Ghita, 22087 Hamburg (DE); Riedel, Heidi, 22083 Hamburg (DE); Ruppert, Stephan, 20259 Hamburg (DE); Eckers, Lorenz, 21255 Tostedt (DE); Kallmayer, Volker, 22525 Hamburg (DE)

(57) **Zusammenfassung**

Die vorliegende Erfindung betrifft eine kosmetische Zubereitung, einen Spender für kosmetische Zubereitungen mit einem die kosmetische Zubereitung enthaltenden, im wesentlichen zylindrischen Behälter, der bodenseitig einen unter dem Druck der Außenatmosphäre an einer Behälterinnenwand gleitverschieblichen Nachlaufkolben aufweist und an seinem oberen Ende ein zu dem Behälter gleitverschiebliches Kopfstück trägt, welches einen mit dem Behälter kommunizierend verbindbaren Ausgabekanal für das Produkt aufweist und auf eine handbetätigbare Fördereinrichtung mit einer volumenveränderlichen Förderkammer für das Produkt einwirkt.

## Beschreibung

Die vorliegende Erfindung betrifft ein Kosmetikum welches sich zusammensetzt aus einem Spender und einer Zubereitung mit empfindlichen Inhaltsstoffen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsyndets) angeboten. Hautpflegeprodukte, in der Regel Cremes, Salben oder Lotionen, dienen meist der Befeuchtung und Rückfettung der Haut sowie der Versorgung der Haut mit kosmetischen oder dermatologischen Wirkstoffen (z.B. Vitaminen, Antioxidantien, UV-Lichtschutzfiltern). Dies ist gerade dann wichtig, wenn das natürliche Regenerationsvermögen der Haut nicht ausreicht, um die durch Umwelteinflüsse, mechanische oder chemische Reize, Sonnenlicht und Wind entstandenen Belastungen (die u.a. die Hautalterung beschleunigen) und Schäden der Haut auszugleichen.

Herkömmliche kosmetische Zubereitungen werden je nach Viskosität und Anwendungszweck in unterschiedlichen Verpackungsmitteln aufbewahrt und dargereicht. Neben den seit langem bekannten Tuben, Flaschen, Aerosol-Sprays etc., finden in jüngerer Zeit zunehmend auch mechanische Pumpspender Verwendung. Mit diesen können nicht nur niedrigviskose wässrige oder wässrig-alkoholische Zubereitungen appliziert werden; sie werden immer häufiger zum Auftragen/Dosieren von Milchen und Lotionen auf Emulsionsbasis eingesetzt.

Gegenüber beispielsweise Flaschen und Tuben haben Pumpspender als Aufbewahrungsund Auftragungsvorrichtung für kosmetische und/oder dermatologische Zubereitungen den Vorteil, dass sich, bedingt durch das Fördervolumen der Pumpe des Spenders, die Zubereitungen besonders einfach dosieren lassen, so dass die Entnahmemenge der Zubereitung bereits vom Hersteller auf die für die Anwendung benötigte Menge eingestellt werden kann. Über- und Unterdosierungen durch den Anwender lassen sich so vermeiden.

Ein weiterer Vorteil von Pumpspendern gegenüber herkömmlichen Verpackungsmitteln besteht in dem Umstand, dass sich die Zubereitungen relativ sauber und hygienisch aus dem Vorratsgefäß entnehmen lassen. Ein direkter Kontakt der in dem Vorratsgefäß verbleibenden Restzubereitung mit Körperoberflächen (z.B. den Händen) und eine dadurch bedingte Kontaminierung mit Keimen (z.B. Bakterien, Pilzen, etc.) wird wirkungsvoll vermieden, da die Zubereitung unter Druck aus dem Vorratsgefäß herausgeschleudert wird.

Gegenüber den seit Jahren bekannten Aerosoldosen haben Pumpspender den großen Vorteil, dass das Gefahrenpotential, welches aus einer möglichen Beschädigung eines Druckgasbehälters (Aerosoldose) resultiert, vollständig vermieden wird. Da Pumpspender ohne den Einsatz die Ozonschicht gefährdender oder brennbarer Treibgase als Fördermedium auskommen, ist ihr Einsatz besonders sicher und umweltfreundlich. Nicht zuletzt sind Pumpspender preisgünstiger und umweltschonender herzustellen und zu entsorgen.

Es wurden daher in der Vergangenheit eine Vielzahl mechanischer Pumpspender (im Rahmen dieser Beschreibung auch Spender genannt) für kosmetische Zubereitungen entwickelt, mit denen sich auch höher viskose Formulierungen applizieren lassen.

So sind Spender mit gleitverschieblichen Nachlaufkolben und handbetätigbarer Fördereinrichtung mit einer volumenveränderlichen Förderkammer als ambulante Vorratsbehälter in einer Vielzahl von Anwendungsbeispielen bekannt, z. B. für die Körperpflege, in der Medizin für die Applikation von Medikamenten oder auch bei der Bereitstellung pastöser Lebensmittel im Handel. Entsprechend vielgestaltig ist auch die Ausgestaltung der für die Bereitstellung der sehr unterschiedlichen pastösen Massen verwendeten Spender, vor allen in Bezug auf ihren unmittelbaren Förder -und Handhabungsmechanismus.

Ein solcher Spender ist beispielsweise aus der EP-A-0 230 252 bekannt. Bei diesem vorbekannten Spender weist die handbetätigbare Fördereinrichtung einen Förderkolben auf, durch welchen das Volumen der Förderkammer veränderbar ist. Der Förderkolben ist mit einem Rohrstück verrastet, welches einstückig an dem Kopfteil ausgeformt ist. Bei der Benutzung des Spenders wird das Kopfstück von einer Ausgangslage durch Handbetätigung in axialer Richtung in Richtung auf den Behälter verschoben.

Diese Verschiebebewegung führt direkt zu einer Gleitbewegung des Förderkolbens entlang der Innenwandung der Förderkammer unter Verringerung des Volumens derselben. Der hierbei aufgebaute Innendruck in der Förderkammer führt zunächst zur Öffnung eines in dem Förderkolben ausgebildeten ellipsenförmig eine Durchlassöffnung des Förderkolbens abdeckenden Rückschlagventils, durch welches dann das pastöse Produkt bei einer weiteren Verringerung des Volumens der Förderkammer in Richtung auf den Ausgabekanal zur Entnahme an einer Produktabgabeöffnung, die an dem Kopfteil ausgebildet ist, gefördert wird.

Bei dem vorbekannten Spender muss in der Förderkammer zunächst ein die Verschlusskräfte des Rückschlagventils überwindender Innendruck aufgebaut werden.

Auch das Fördern der pastösen Masse durch das Rückschlagventil führt zu einem Druckverlust, welcher insofern nachteilig ist, als dass zur Kompensation dieses Druckverlustes erhöhte Druckkräfte aufgewendet werden müssen, um das Kopfteil in Richtung auf den Behälter axial zu verschieben.

Die Schrift WO 03/004374 offenbart einen Spender, der dahingehend verbessert ist, dass eine Betätigung mit geringeren Betätigungskräften möglich ist. Dabei umfasst die Fördereinrichtung ein zu dem Behälter und dem Kopfstück längsverschiebliches Förderelement, welches einen in der Förderkammer gleitverschieblichen Förderkolben aufweist, der mit einem Förderschaft verbunden ist, welcher einen Förderkanal umfänglich umgibt, der eine mit der Förderkammer kommunizierende Förderkanaleinlassöffnung und eine Förderkanalauslassöffnung aufweist, die durch eine Verschiebebewegung des Förderelementes relativ zu dem Kopfstück in eine Stellung bringbar ist, in der sich die Förderkanalauslassöffnung zu dem Ausgabekanal öffnet.

Mechanische Pumpspender des Standes der Technik weisen ferner die folgenden Nachteile auf:
Die kosmetische Zubereitung ist in dem Vorratsbehälter nicht ausreichend von der Umwelt abgeschirmt. Das bei der Anwendung des Kosmetikums aus dem Vorratsgefäß entnommene Volumen an Zubereitung muss durch Außenluft ersetzt werden, wodurch Feuchtigkeit (Wasser) und Sauerstoff in den Vorratsbehälter eingetragen werden und mit der Zubereitung in Berührung kommen. Dieser Luft-Eintrag in das Vorratsgefäß ist bei keinem Pumpspender zu vermeiden. Problematisch ist der Kontakt mit Luft für die im Vorratsgefäß aufbewahrte Zubereitung insbesondere dann, wenn die Formulierung einen oder mehrere Inhaltsstoffe (insbesondere Wirkstoffe) enthält, die
   - oxidationsempfindlich gegenüber Sauerstoff oder Luft und/oder
   - hydrolyseempfindlich gegenüber Wasser oder Luftfeuchtigkeit sind,
   - eine Temperaturänderung der Zubereitung unter Einwirkung von Wasser, Luftfeuchtigkeit und/oder Sauerstoff bewirken,
   - ihre physiologische Wirksamkeit unter Einwirkung von Wasser, Luftfeuchtigkeit und/oder Sauerstoff einbüßen und/oder
   - die Geruchsrezeptoren von Menschen und/oder Tieren reizen, beispielsweise Duftund/oder Parfumstoffe und/oder Repellentien enthalten.

Enthält eine kosmetische oder dermatologische Zubereitung mindestens einen derartigen Inhaltsstoff, verliert und/oder ändert sie über einen längeren Aufbewahrungszeitraum im Pumpspender zunehmend ihre Wirksamkeit bzw. ihre vom Hersteller vorgegebene Zusammensetzung.

Erfahrungsgemäß stellen die durch Volumenaustausch in das Vorratsgefäß eingetragenen Luft- d.h. insbesondere Sauerstoff- und Wassermengen eine untergeordnete Rolle im Vergleich zu der Luft- bzw. Sauerstoff und Feuchtigkeitskontamination, die daraus resultiert, dass das Vorratsbehältnis an sich nicht ausreichend von der Umwelt abgeschirmt ist. Besonders kritisch ist die Austrittsöffnung, da hier Produkt ungeschützt der Umgebung ausgesetzt ist. Im Extremfall wird es dem Verwender unmöglich sein, unzersetztes Produkt zu entnehmen, da immer das zur nächsten Entnahme anstehende Produktvolumen der stärksten Belastung durch Umgebungssauerstoff oder Luftfeuchtigkeit ausgesetzt ist. Auch Stopfen, die nach der Verwendung die Austrittsöffnung verschließen sollen, lösen das Problem nicht angemessen, da hierbei immer Produkt seitlich herausgequetscht wird. Durch den dauerhaften (kontinuierlichen) Kontakt mit "frischem" Sauerstoff bzw. Wasser aus der Umgebungsluft, kommt es über einen längeren Zeitraum zum Wirkungsverlust bzw. einer Änderung der Zusammensetzung der Zubereitung auch im Vorratsgefäß, weshalb Pumpspender nach dem Stande der Technik nicht für Zubereitungen mit den oben genannten Inhaltsstoffen verwendet werden können.

Es war daher die Aufgabe der vorliegenden Erfindung, die Mängel des Standes der Technik zu beseitigen und Kosmetika zu entwickeln, welche die beschriebenen Mängel nicht oder nur in geringem Maße aufweisen. Insbesondere sollte ein Kosmetikum entwickelt werden, mit dem sich mit Hilfe eines mechanischen Pumpspenders auch Zubereitungen mit oxidationsund/oder feuchtigkeitsempfindlichen Inhaltsstoffen in gleich bleibender Qualität aufbewahren und darreichen lassen.

Weiterhin lag der vorliegenden Erfindung die Aufgabe zugrunde, einen Spender der eingangs genannten Art dahingehend zu verbessern, dass eine Betätigung mit geringeren Betätigungskräften möglich ist und darüber hinaus eine Beeinträchtigung des abzugebenden pastösen Produktes durch Oxidation vermindert wird.

Überraschend gelöst werden die Aufgaben durch ein Kosmetikum aus a) einem Spender für pastöse Produkte mit einem eine kosmetische und/oder dermatologische Zubereitung enthaltenden, im Wesentlichen zylindrischen Behälter (1), der bodenseitig einen unter dem Druck der Außenatmosphäre an der Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet dass die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet, und
b) einer kosmetische Zubereitung enthaltend einen oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen.

Die Aufgaben werden ferner überraschend gelöst durch die Verwendung eines Spenders für pastöse Produkte mit einem eine kosmetische und/oder dermatologische Zubereitung enthaltenden, im Wesentlichen zylindrischen Behälter (1), der bodenseitig einen unter dem Druck der Außenatmosphäre an der Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet dass die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet,
zur Aufbewahrung und Darreichung kosmetischer und/oder dermatologischer Zubereitungen enthaltend einen oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen.

Nach Produktentnahme aus den beschriebenen Spendern wird erfindungsgemäß vorteilhaft die Förderkanalauslassöffnung geschlossen, wobei das Füllgut nicht nach außen, sondern nach innen gedrückt wird und so vor der Umgebung geschützt wird.

Im Rahmen dieser Offenbarung bezieht sich der Begriff "erfindungsgemäß" sowohl auf das Kosmetikum, den Spender und die Zubereitung an sich als auch auf Verwendungen derselben.

Im Rahmen dieser Offenbarung bedeutet pastöse Zubereitung erfindungsgemäß, dass die Zubereitung eine Viskostät von 500 bis 20.000 mPas aufweist (gemessen mit Hilfe eines Viskosimeters des Typs Viskotester VT 02 der Gesellschaft Haake mit den folgenden Messparametern: Temperatur: 25°C, Drehkörperdurchmesser 24 mm, Rotorgeschwindigkeit 62,5 1/min).

Überraschend war es auch für den Fachmann, dass sich die erfindungsgemäßen Zubereitungen mit einer reduzierten Menge an Konservierungsstoffen gegen den Befall durch Mikroorganismen wirkungsvoll schützen ließen.

Erfindungsgemäß vorteilhaft enthält das erfindungsgemäße Kosmetikum bzw. der erfindungsgemäß verwendete Spender in der Zubereitung ein oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen, jeweils in einer Gesamtkonzentration von 0,01 bis 5 Gew.-%, bevorzugt in einer Konzentration von 0,05 bis 3 und ganz besonders bevorzugt in einer Konzentration von 0,1 bis 1 Gew.-%, jeweils bezogen auf das Gesamtgewicht der Zubereitung.

Erfindungsgemäß vorteilhaft ist das erfindungsgemäße Kosmetikum bzw. der erfindungsgemäß verwendete Spender dadurch gekennzeichnet, dass ein oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen, gewählt werden aus der Gruppe der Antioxidantien, Vitamine, Enzyme, Coenzyme, Parfumstoffe, Duftstoffe, Repellentien, Selbstbräuner, ungesättigte Lipide Öle, Fette, Wachse, Polyphenole, Enzyme, Flavonoide und Isoflavonoide, Lignane, Polyole, Polyethylenglykole sowie der Pflanzenextrakte, die einen oder mehrere der vorgenannten Wirkstoffe enthalten.

Dabei ist es erfindungsgemäß bevorzugt, wenn das erfindungsgemäße Kosmetikum bzw. der erfindungsgemäße Spender in der Zubereitung ein oder mehrere Inhaltsstoffe gewählt aus der Gruppe bestehend aus Vitamin A und seinen Derivaten; Vitamin B und seinen Derivaten; Vitamin C und seinen Derivaten; Vitamin E und seinen Derivaten; Vitamin F; Polyphenole, Ubichinon Q 10 (auch in seiner reduzierten Form); 2,6-Ditert.-butyl-4-methylphenol; Dihydroxyaceton; Niacinamid; Pantothensäure und ihre Salze; Panthenol; γ-Oryzanol; Biotin; Kreatin, Kreatinin; Subtilisin; α-Glycosylrutin; Allantoin; Tannin; Azulen; Bisabolol; Glycyrrhizin; Hamamelin; Harnstoff; Genistin; Genistein; Daidzin; Daidzein; Carnitin und seine Derivate und Octadecendicarbonsäure
als Wirkstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen.

### I. Die erfindungsgemäße Zubereitung

Erfindungsgemäß vorteilhaft enthält das erfindungsgemäße Kosmetikum bzw. der erfindungsgemäß verwendete Spender in der Zubereitung auch einen oder mehrere der folgenden Inhaltsstoffe, die je nach ihrer chemischen Natur, ebenfalls unter die erfindungsgemäßen Wirkstoffe fallen können:

Als erfindungsgemäß vorteilhafte UV-Lichtschutzfilter können beispielsweise die folgenden Verbindungen eingesetzt werden:

### Anorganische Pigmente

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wässriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| **Handelsname** | **Coating** | **zusätzliche Bestand- teile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2 Gew.-% Dimethicone | BASF |
| Z- Cote | / | BASF |
| ZnO NDM | 5 Gew.-% Dimethicone | H&R |
| MZ 707M | 7 Gew.-% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

### Organische Pigmente

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

### Weitere UV-Lichtschutzfilter

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'tetrasulfonsäure-bis-natriumsalz mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Weitere vorteilhafte UV-A-Filtersubstanzen sind Hydroxybenzophenone, die sich durch die folgende Strukturformel auszeichnen: 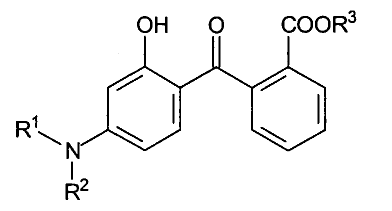 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 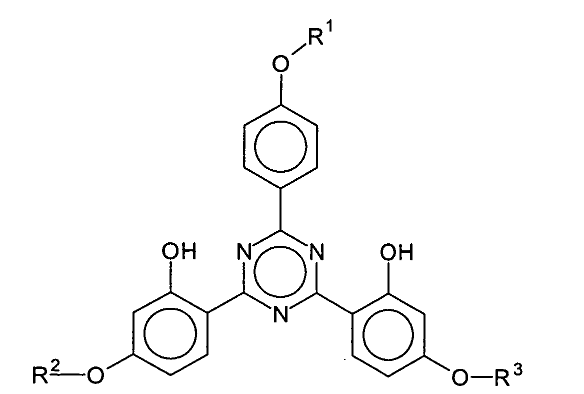 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen; welche das Strukturmotiv 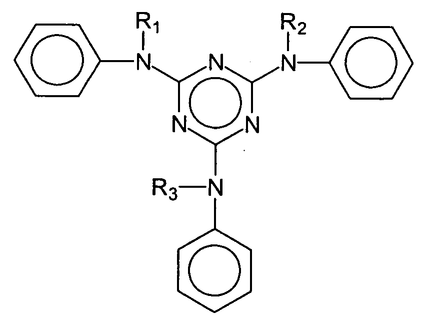 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 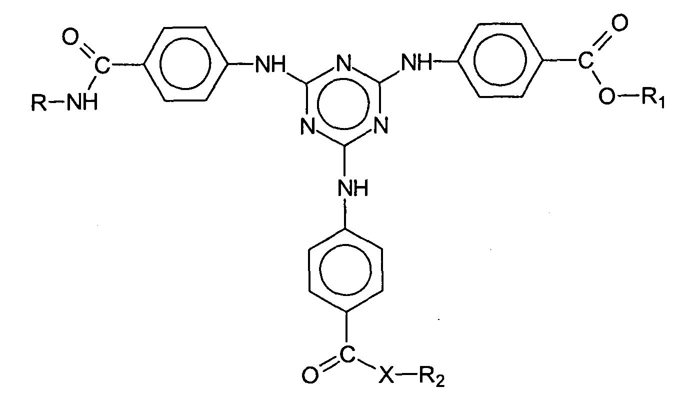 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 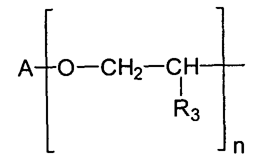 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein
Wasserstoffatom, ein Alkalimetallatom, eine Ammoniumgruppe oder eine Gruppe der Formel 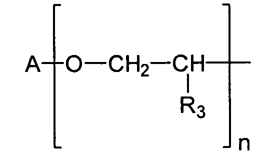 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
- R₃: ein Wasserstoffatom oder eine Methylgruppe darstellt,
- n: eine Zahl von 1 bis 10 darstellt,
- wenn X: ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 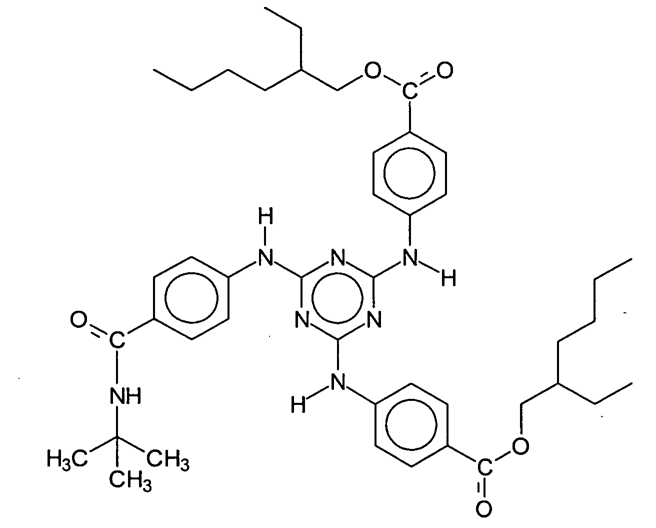 wiedergegeben wird, welches im Folgenden auch als Dioctylbutylamidotriazon (INCl: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 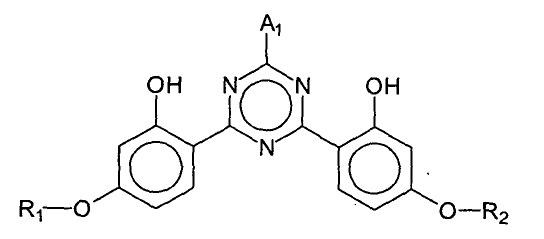 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethylcarboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylenbis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy)disiloxanyl]propyl]-phenol (CAS-Nr.: 155633-54-8) mit der INCl-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)-ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

### Vitamine und Vitaminderivate

Hierzu zählen unter anderem die Vitamine A, B₁₋₆, B₁₂, C, D, E, F, H, K und PP sowie deren Derivate. Diese können erfindungsgemäß vorteilhaft in einer Konzentration von 0,001 bis 10 Gew.-%, bevorzugt 0,05 - 7 Gew.-%, insbesondere bevorzugt 0,5 - 5 Gew.-%, jeweils bezogen auf das Gesamtgewicht der Zubereitung, enthalten sein.

Als erfindungsgemäß bevorzugte Vitaminderivate werden dabei Retinylpalmitat, Ascorbylglucosid, Tocopherylacetat, Tocopherylpalmitat, Niacinamid, Panthenol eingesetzt.

Erfindungsgemäß vorteilhaft ist der Einsatz von Vitaminen und deren Derivaten in einer Konzentration von 0,05 bis 1 Gewichts- %, bevorzugt in einer Konzentration von 0,01 bis 0,5 Gew.-% und ganz besonders bevorzugt in einer Konzentration von 0,05 bis 0,2 Gew.-%, jeweils bezogen auf das Gesamtgewicht der Zubereitung.

### Enzyme

Das erfindungsgemäße Kosmetikum bzw. der erfindungsgemäß vorteilhaft verwendete Spender enthält vorteilhaft Enzyme. Als Enzyme können dabei vorteilhaft im Sinne der vorliegenden Erfindung Lipasen, Esterasen, Proteasen und andere Hydrolasen eingesetzt werden, wobei Lipasen erfindungsgemäß bevorzugt sind.

Als erfindungsgemäße Enzyme können vorteilhaft Enzymextrakte aus *Alcaligenes sp.* (z.B. von der Firma Amano Enzyme Europe Ltd.), *Aspergillus niger* (z.B. von den Firmen Amano Enzyme Europe Ltd. oder Fluka), *Candida cylindracea* (z.B. von den Firmen Sigma, Aldrich, Fluka, Amano Enzyme Europe Ltd., Boeringer-Mannheim), *Candida* lipolytica (z.B. von den Firmen Amano Enzyme Europe Ltd., Fluka), *Chromobacterium viscosium* (z.B. von den Firmen Sigma), *Humicola laguninosa* (z.B. von der Firma Amano Enzyme Europe Ltd.), *Mucor miehei* (z.B. von den Firmen Amano Enzyme Europe Ltd., Novo), *Penecillium* roqueforti (z.B. von der Firma Fluka), *Porcine pancreas* (z.B. von den Firmen Sigma, Aldrich, Fluka, Amano Enzyme Europe Ltd., Boeringer-Mannheim), *Pseudomonas aegruginosa* (z.B. von der Firma Amano Enzyme Europe Ltd.), *Pseudomonas* fluorescens (z.B. von den Firmen Amano Enzyme Europe Ltd., Fluka), *Pseudomonas* sp.(z.B. von den Firmen Sigma, Boeringer-Mannheim), *Rhizopus delemar* (z.B. von der Firma Sigma, Fluka, Boeringer-Mannheim), *Rhizopus japonicus* (z.B. von den Firmen Amano Enzyme Europe Ltd.), *Rhizopus oryzae* (z.B. von der Firma Amano Enzyme Europe Ltd.), *Rhizopus sp.* (z.B. von den Firmen Amano Enzyme Europe Ltd., Serva) und/oder Weizenkeim (z.B.von der Firma Sigma) eingesetzt werden.

Dabei sind als Enzym-Extrakte Lipasen aus Schweinepankreas (*Porcine pancreas*), *Aspergillus niger, Chromobacterium viscosium, Geotrichum candidum, Penecillium camberti, Penecillium roqueforti* erfindungsgemäß bevorzugt.

Erfindungsgemäß ganz besonders bevorzugt sind Lipasen der Art *Mucor miehei* (z.B. Lipozyme® von der Firma Novo Nordisk ), *Humicola laguninosa, Pseudomonas aegruginosa, Pseudomonas fluorescens, Pseudomonas sp., Candida cylindracea* und/oder *Candida lipolytica.*

Erfindungsgemäß vorteilhaft liegen ein oder mehrere Enzyme in Form einer wässrigen Lösung vor. Deren Gehalt an einem oder mehreren Enzymen beträgt erfindungsgemäß vorteilhaft von 0,1 bis 5 Gewichts- %, bevorzugt von 1 bis 5 Gewichts- % und ganz besonders bevorzugt von 1 bis 2 Gew.-%, jeweils bezogen auf das Gesamtgewicht der Zubereitung.

### Antioxidantien

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Satz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptidund Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

### Antifaltenwirkstoffe, Pflanzenextrakte und andere Wirkstoffe

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Lignane, Taurin und/oder β-Alanin.

Erfindungsgemäß vorteilhaft können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin sowie Pflanzenwirkstoffe wie Azulen und Bisabolol, Glycyrrhizin, Hamamelin und Pflanzenextrakte wie Kamille, Aloe Vera, Hamazelis, Süßholzwurzel.

Die Menge der vorgenannten Wirkstoffe (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

### Selbstbräuner

Als Selbstbräuner werden erfindungsgemäß vorteilhaft unter anderem eingesetzt: Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon), 2-Hydroxy-1,4-naphtochinon (Lawson).

Besonders bevorzugt im Sinne der Erfindung ist das 1,3-Dihydroxyaceton (DHA). Von Vorteil ist dabei eine Konzentration von 0,5 bis 10 Gew.-% 1,3-Dihydroxyaceton und besonders eine Konzentration von 1 bis 7 Gew.-%, bezogen auf das Gesamtgewicht der Zubereitung.

### Depigmentiermittel

Als erfindungsgemäß vorteilhafte Depigmentierungsmittel können beispielsweise Dicarbonsäuren wie 8-Hexadecen-1,16-dicarbonsäure (Dioic acid, CAS-Nummer 20701-68-2), Kojisäure, Ascorbinsäure und Azelainsäure sowie deren Derivate eingesetzt werden.

Erfindungsgemäß vorteilhaft beträgt die Gesamtkonzentration an Depigmentierungsmitteln in der entsprechenden Zubereitung 0,001 - 10 Gew.-%, bevorzugt 0,005 - 8 Gew.-%, insbesondere 0,05 - 5 Gew.-%.

### Repellentien

Erfindungsgemäß vorteilhaft können eingesetzt werden:
N,N-Diethyl-3-methylbenzamid (DEET), Dimethylphthalat (Palatinol M, DMP), 2,3;4,5-bis-(2-Butylen)-tetrahydro-2-furaldehyd (MGK-Repellent 11), Butopyronoxyl (Indalone), N,N-Caprylsäurediethylamid (Repellent 790), o-Chlor-N,N-diethylbenzamid in Mischung mit N,N-Diethylbenzamid (Kik-Repellent), Dimethylcarbat (Dimalone), Di-n-propylisocinchomeronat (MGK-Repellent 326), 2-Ethylhexan-1,3-diol (Rutgers 612), N-Octyl-bicycloheptendicarboximid (MGK 264 Insecticidesynergist), Piperonyl-butoxid (PBO) und, erfindungsgemäß besonders bevorzugt, 3-(N-n-Butyl-N-acetyl-amino)propionsäureethylester (auch als Repellent 3535 bezeichnet) sowie 1-Piperidincarbonsäure-2-(2-hydroxyethyl)-1-methylpropylester (Bayrepel® ).

Erfindungsgemäß vorteilhaft ist ein Gehalt an Repellentien von 0,005 bis 70,0 Gew.-% eingesetzt, insbesondere 0,01 bis 50,0 Gew.-% und ganz besonders bevorzugt 3 bis 15 Gew.-% bezogen auf das Gesamtgewicht der Zubereitung.

### Wasserfreie Polyole

Es ist erfindungsgemäß bevorzugt, wenn die Polyalkohole gewählt werden aus der Gruppe der Substanzen mit einem Molekulargewicht zwischen 200 und 600 g/mol, beispielsweise Polyethylenglycol(200) (=PEG-4), Polyethylenglycol(300) (=PEG-6), Polyethylenglycol(400) (=PEG-8), Polyethylenglycol(1000) (=PEG-5) und Polyethylenglycol(1200) (=PEG-6). Die erfindungsgemäß ganz besonders bevorzugten Polyalkohole sind die Verbindungen Polyethylenglycol(400) (=PEG-8), Polyethylenglycol(1000) (=PEG-5) und Polyethylenglycol(1200) (=PEG-6).

Erfindungsgemäß bevorzugt ist eine Polyalkoholkonzentration von 5 40 bis 90 Gew.-% und besonders bevorzugt von 40 bis 85 Gew.-%, bezogen auf das Gesamtgewicht der Zubereitung.

Unter "wasserfrei" werden erfindungsgemäß Polyole verstanden, deren Wasserkonzentration kleiner als 5 Gew.-% und bevorzugt kleiner als 3 Gew.-%, jeweils bezogen auf das Gewicht des Polyols ist.

Polyol-haltige Zubereitungen werden erfindungsgemäß vorteilhaft insbesondere als nahezu wasserfreie Zubereitungen angeboten, damit sie bei der Anwendung und dem Kontakt mit Wasser als sogenannte "Selbstwärmende" (da sie Kontakt mit Wasser Wärme freisetzen) Kosmetika bzw. Dermatika dargereicht werden können.

### Farb- und Effektstoffe

Als erfindungsgemäß vorteilhafte pigmentäre Farbstoffe können alle aus der entsprechenden Positivliste der Kosmetikverordnung bzw. der EG-Liste kosmetischer Färbemittel aufgelisteten Verbindungen eingesetzt werden. In den meisten Fällen sind sie mit den für Lebensmittel zugelassenen Farbstoffen identisch. Neben Farbpigmenten können die erfindungsgemäßen Zubereitungen auch weitere Farbstoffe enthalten. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | Grün |
| Acid Green 1 | 10020 | Grün |
| 2,4-Dinitrohydroxynaphthalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hy- | 12490 | Rot |
| droxy-5"-chloro-2",4"-dimethoxy-2-naphthoesäureanilid Disperse Yellow 16 | 12700 | Gelb |
| 1-(4-Sulfo-1 -phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin- | 14700 | Rot |
| 4-sulfosäure 2-(4-Sulfo-1-naphthylazo)-1-naphthol-4-sulfosäure | 14720 | Rot |
| 2-(6-Sulfo-2,4-xylylazo)-1 -naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1 -phenylazo)-2- | 15525 | Rot |
| hydroxynaphthalin 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1 -phenylazo)-2-hydroxy- | 15865 | Rot |
| naphthalin-3-carbonsäure 1-(2-Sulfo-1 -naphthylazo)-2-hydroxynaphthalin-3-carbonsäure | 15880 | Rot |
| 1-(3-Sulfo-1 -phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1 -naphthylazo)-2-naphthol-3,6-disulfosäure | 16185 | Rot |
| Acid Orange 10 | 16230 | Orange |
| 1-(4-Sulfo-1 -naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2 -phenylazo- 1 -naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxypyrazolon-3-carbonsäure | 19140 | Gelb |
| Pigment Yellow 16 | 20040 | Gelb |
| 2,6-(4'-Sulfo-2", 4"-dimethyl)-bis-phenylazo)1,3-dihydroxybenzol | 20170 | Orange |
| Acid Black 1 | 20470 | Schwar z |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd (C₃₀) | 40820 | Orange |
| trans-Apo-8'-Carotinsäure (C₃₀)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenylcarbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | Blau |
| Acid Green 9 | 42100 | Grün |
| Diethyl-di-sulfobenzyl-di-4-amino-2-chlor-di-2-methylfuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfonsäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 -phenylazo- 1 -naphthol-3,6-disulfosäure, Aluminiumsalz der 4-(4-Sulfo-1 -phenylazo)-1 -(4-sulfophenyl)-5-hydroxy-pyrazolon-3-carbonsäure, 4'-[(4"-Sulfo-1"-phenylazo)-7'-sulfo-1'-naphthylazo]-1-hydroxy-8-acetyl-aminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigodisulfosäure, 4,4'-Dimethyl-6,6'-dichlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Erfindungsgemäße Titandioxide, die sowohl in der Kristallmodifikation Rutil als auch Anatas vorliegen können, sind im Sinne der vorliegenden Erfindung vorteilhaft oberflächlich behandelt ("gecoatet"), wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Zirkoniumoxid (ZrO₂) oder Eisenoxid (Fe₂O₃). Diese anorganischen Oberflächenbeschichtungen können allein, in Kombination und/oder in Kombination mit organischen Beschichtungsmaterialien vorkommen.
Hierzu werden Oxide, Oxidhydrate oder Phosphate beispielsweise der Elemente Al, Si, Zr in dichten Schichten auf die Pigmentoberfläche aufgefällt.
Die anorganische Nachbehandlung geschieht im allgemeinen in einer wässrigen Suspension des Pigmentes durch Zugabe löslicher Nachbehandlungschemikalien, wie z.B. Aluminiumsulfat, und anschließende Ausfällung des im neutralen Bereich schwerlöslichen Hydroxides durch gezielte Einstellung des pH-Wertes mit Natronlauge.
Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z.B. die von Sun Chemical erhältlichen Titandioxid Typen C47-051 und C47- 5175. Weiterhin bevorzugte Pigmente sind Titandioxide, die mit Aluminium- und / oder Siliziumoxiden gecoated sind, wie z.B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Ferner kann es erfindungsgemäß vorteilhaft sein Perlglanzpigmente einzusetzen.
Dazu zählen natürliche Perlglanzpigmente, wie z. B.
■ "Fischsilber" (Guanin/Hypoxanthin-Mischkristalle aus Fischschuppen) und
■ "Perlmutt" (vermahlene Muschelschalen),
monokristalline Perlglanzpigmente wie z. B. Bismuthoxychlorid (BiOCl),
Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| ***Gruppe*** | Belegung / Schichtdicke | Farbe |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtön e |
| | TiO₂ / Cr₂O₃ | grün |
| | TiO₂ / Berliner Blau | tiefblau |
| | TiO₂ / Carmin | rot |

Besonders bevorzugt sind die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Die erfindungsgemäß besonders bevorzugten pigmentären Farbstoffe sind die aufgeführten blauen Pigmente wie z.B. INCI: Cl 77007, Outremer Supercosmetique W 6803 von Les Colorants Wackherr, INCI: Cl 77891 + Mica + Silica, Timiron Splendid Blue von Merck.

Erfindungsgemäß bevorzugt sind ferner mit Titandioxid und Siliciumdioxid beschichteter Glimmer, z.B. INCI: Mica + Cl 77891 + Silica, Timiron Arctic Silver von Merck, INCl: Mica + Cl 77891, Timiron Gleamer Flake MP-45 von Merck; mit Titandioxid und Zinnoxid beschichtetes Aluminiumoxid oder Siliciumdioxid, z.B.INCI: Silica + Cl 77891 + Tin Oxide, Xirona Magic Mauve von Merck; z.B.INCI: Alumina + Cl 77891 + Tin Oxide, Xirona Silver, mit Titandioxid und Berliner Blau bschichteter Glimmer, z.B. INCI: Mica + Cl 77891 + Cl 77510, Colorona Light Blue und Colorona Dark Blue von Merck.

### Perlglanz:

Bevorzugt ist auch der Einsatz Perlglanzmitteln auf Basis von Dialkylethern, die bei 30°C fest sind und z.B: folgende Formel haben: R-O-R'. Dabei können R und R'gleich oder unterschiedlich sein; geradkettige oder verzweigte, gesättigte oder ungesättigte Alkylradikale sein. Diese können aus 12 bis 30 Kohlenstoffatomen bestehen, bevorzugt aus 14 bis 24 Kohlenstoffatomen. Besonders bevorzugt bestehen R und R' einem Stearylradikal (z.B. INCI: Distearyl Ether, Cutina STE von Cognis).
Die verwendeten Dialkylether sind bei einer Konzentration von über 0,1% bei 25°C nicht in Wasser löslich.

Vorzugsweise könne auch acylierte Radikale, die aus einer Fettsäurekette mit 8 bis 30 Kohlenstoffatomen bestehen, eingesetzt werden.
Pro acyliertem Derivat ist mindenstens eine RC(=O)- Gruppe enthalten, wobei R eine Fettsäurekette mit mit 8 bis 30 Kohlenstoffatomen ist.
Insbesondre Ethylenglycol Monostearate und Ethylenglycol Distearate, z.B. INCI: Glycol Distearate, Cutina AGS von Cognis, INCI: Aqua + Glycol Distearate + Glycerin + Laureth-4 + Cocamidopropyl Betaine, Euperlan PK 3000 OK von Cognis, INCI: PEG-3 Distearate, Cutina TS von Cognis.
Diese werden erfindungsgemäß vorteilhaft in einer Konzentration zwischen 0,5% und 2% eingesetzt.

### Trübungsmittel:

Erfindungsgemäß vorteilhaft möglich ist auch der Einsatz von Trübungsmitteln. Bevorzugt werden die Natriumsalze eines Polymers aus Styrol mit einem Monomer bestehend aus Acrylsäure, Methacrylsäure oder einem anderen Olefin und einer ihrer Ester, z.B. INCI: Sodium Styrene/Acrylates Copolymer, Acusol OP 301 von Rohm & Haas.
Diese werden erfindungsgemäß vorteilhaft in einer Konzentration zwischen 0,5% und 2% eingesetzt.

Auch Glitter- und Glitzerpartikel können erfindungsgemäß vorteilhaft in mindestens einer der Zubereitungen enthalten sein.

Erfindungsgemäß vorteilhaft können die erfindungsgemäßen Zubereitungen ein oder mehrere Tenside enthalten, wobei sowohl anionische, kationische, nichtionische und zwitterionische Tenside eingesetzt werden können.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyloder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Erfindungsgemäß bevorzugt sind Alkylsulfate oder Alkylethersulfate bzw. eine Tensidkombinationen aus Alkylethersulfaten mit amphoteren oder nichtionischen Tensiden, wobei eine Tensidkombination aus aus Alkylethersulfaten mit Alkylamidopropylbetainen oder Alkylamphoacetaten oder Alkylpoyglucosiden besonders bevorzugt ist. Besonders bevorzugt werden auch Kombinationen aus Alkylethersulfaten mit Alkylamidopropylbetainen oder Alkylamphoacetaten und Acylglutamaten.

Es ist vorteilhaft im Sinn der vorliegenden Erfindung, wenn der Gehalt an einem oder mehreren waschaktiven Tensiden in der entsprechenden Zubereitung aus dem Bereich von 1 bis 30 Gew.-%, bevorzugt von 5 bis 25 Gew.-%, ganz besonders vorteilhaft von 10 bis 20 Gew.-% gewählt wird, jeweils bezogen auf das Gesamtgewicht der Zubereitung.

Tensid-haltige Zubereitungen können erfindungsgemäß vorteilhaft als Reinigungszubereitungen eingesetzt werden. Der Gehalt an Tensiden in den erfindungsgemäßen Zubereitungen kann erfindungsgemäß vorteilhaft aber auch zum Aufschäumen der Zubereitungen verwendet werden (Ausführungsform: Schaumspender).

Erfindungsgemäß vorteilhaft können die erfindungsgemäßen Zubereitungen Polysorbate enthalten. Polysorbate stellen eine Verbindungsklasse dar, die sich vom Sorbitan, einem aus Sorbit durch Abspaltung zweier Äquivalente Wasser gewonnenem Furanderivat, ableiteten. Die Hydroxylgruppen des Sorbitans sind mit Polyethylenglykolen verethert, deren Enden mit Fettsäuren verestert sein können. Sie lassen sich allgemein durch die Formel 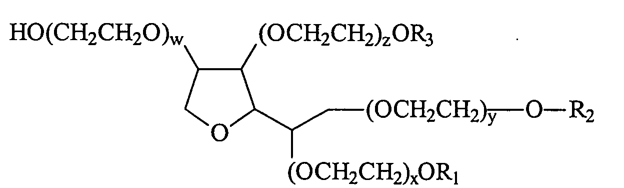 R₁, R₂, R₃ = H, Fettsäurerest
darstellen.

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gew.-% und insbesondere in einer Konzentration von 1,5 bis 2,5 Gew.-%, bezogen auf das Gesamtgewicht der Zubereitung einzeln oder als Mischung mehrer Polysorbate, eingesetzt.

### Hydrokolloide

Die erfindungsgemäßen Zubereitungen enthalten erfindungsgemäß vorteilhaft ein oder mehrere Hydrokolloide. "Hydrokolloid" ist die technologische Kurzbezeichnung für die an sich richtigere Bezeichnung "hydrophiles Kolloid". Hydrokolloide, auch Verdicker oder Gelbildner genannt, sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

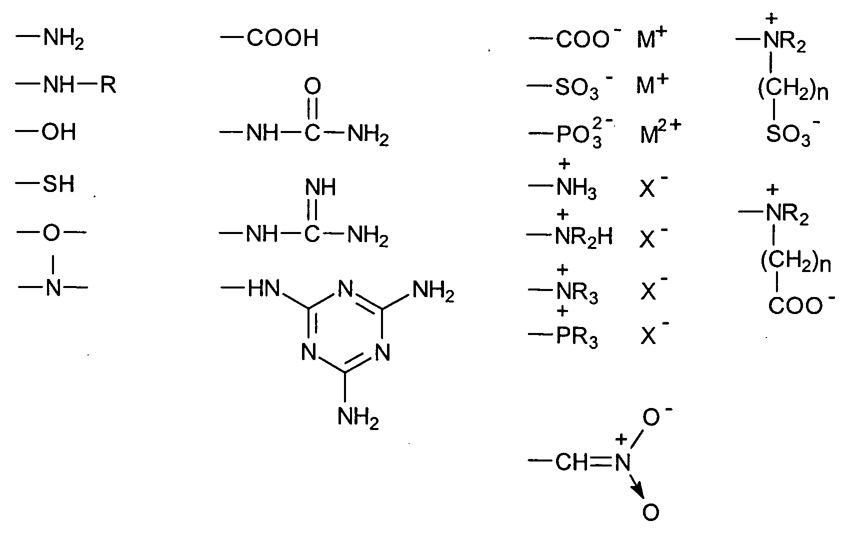

Die Gruppe der kosmetisch und dermatologisch relevanten Hydrokolloide läßt sich wie folgt einteilen in:
organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, organische, abgewandelte Naturstoffe, wie z. B. Carboxymethylcellulose und andere Celluloseether, Hydroxyethyl- und -propylcellulose und dergleichen, organische, vollsynthetische Verbindungen, wie z. B. Polyacryl- und Polymethacryl-Verbindungen, Vinylpolymere, Polycarbonsäuren, Polyether, Polyimine, Polyamide, anorganische Verbindungen, wie z. B. Polykieselsäuren, Tonmineralien wie Montmorillonite, Zeolithe, Kieselsäuren.

Als erfindungsgemäß vorteilhafte Hydrokolloide werden Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Celluloseether, Hydroxyethyl- und -propyl-cellulosederivate, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 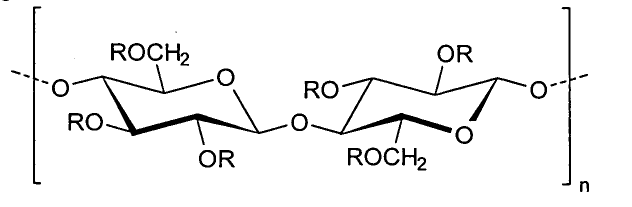 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×106 bis 24×106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, µ, ν, ξ, π, ω, χ. Auch die Art vorhandener Kationen (K⁺, NH₄⁺, Na⁺, Mg²⁺, Ca²⁺) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

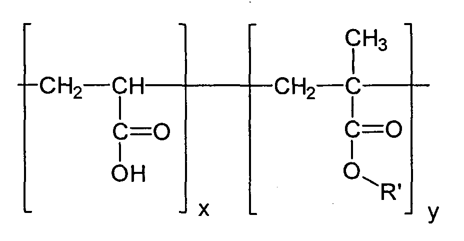

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche beispielsweise unter den Handelbezeichnungen Carbopol® 1382, Carbopol® 981, Carbopol® 980 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCl-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Die erfindungsgemäß besonders bevorzugten Hydrokolloide sind: Acrylates Copolymer (AQUA SF-1), Acrylates/C 10-30 Alkyl Acrylate Crosspolyme (Carbopol ETD 2020), Xanthan Gum (Kelter).

Die erfindungsgemäßen Zubereitungen enthalten vorteilhaft ein oder mehrere Hydrokolloide / Gelbildner in einer Konzentration von 0,1 bis 8 Gew.-%, bevorzugt von 0,2 bis 6 Gew.-% und ganz besonders bevorzugt von 0,3 bis 4 Gew.-%, jeweils bezogen auf das Gesamtgewicht der Zubereitung enthält.

### Filmbildner

Erfindungsgemäß vorteilhaft kann die Zubereitung einen oder mehrere Filmbildner enthalten. Erfindungsgemäß vorteilhafte Filmbildner können dabei aus den in der Tabelle aufgelisteten Verbindungen gewählt werden.

| **Bezeichnung nach INCI** | **CAS-Nummer** | **Polymertyp** | **Beispiel (Handelsname)** |
|---|---|---|---|
| Polyquaternium-2 | CAS 63451-27-4 | Urea, N, N'- bis[3-(dimethylamino)propyl]-, polymer mit 1, 1'-oxybis(2- chloroethan) | Mirapol® A-15 |
| Polyquaternium-5 | CAS 26006-22-4 | Acrylamid, β-Methacryloxyethyltriethyl ammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat® S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SC-230M |
| Polyquaternium-11 | CAS 53633-54-8 | Vinylpyrrolidon/dimethyla minoethyl-Methacrylat-Copolymer/Diethylsulfat-Reaktionsprodukt | Gafquat®755N |
| Polyquaternium-16 | CAS 29297-55-0 | Vinylpyrrolidon/vinylimid azolinummethochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxan-polydimethyldimethylammoniumaceta t-Copolymer | Abil® B 9905 |
| Polyquaternium-22 | CAS 53694-17-0 | Dimethyldiallylammoniu mchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacryl amidopropyltrimethylam moniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/Acryl onitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]-ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |
| Erfindungsgemäß vorteilhafte Filmbildner | | | |

Weitere erfindungsgemäß vorteilhafte Filmbildner stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch nichtionische Poly-N-vinylpyrrolidon/Polyvinylacetat-Copolymere (z.B. Luviskol VA 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Filmbildner eingesetzt werden.

### Komplexbildner

Es ist auch von Vorteil, der erfindungsgemäßen Zubereitung Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

### Lipide

Eine gegebenenfalls gewünschte Ölkomponente der kosmetischen oder dermatologischen Zubereitung im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Dialkylcarbonate, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher ÖI- und Wachskomponenten sind vorteilhaft im Sinne der vorliegenden Erfindung einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 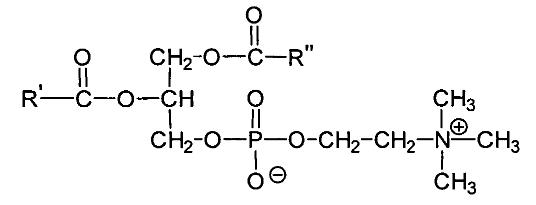 auszeichnen, wobei R' und R" typischerweise unverzweigte aliphatische Reste mit 15 oder 17 Kohlenstoffatomen und bis zu 4 cis-Doppelbindungen darstellen.

### Wässrige Phase

Neben Wasser kann eine erfindungsgemäße wässrige Phase auch wasserlösliche Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyloder -monobutylether, Diethylenglykolmonomethyl- oder -monoethylether und analoge Produkte, ferner Alkohole niedriger C-Zahl, z.B. Ethanol, Isopropanol, 1,2-Propandiol Methylpropandiol und Glycerin.

Erfindungsgemäß vorteilhaft liegt das erfindungsgemäße Kosmetikum bzw. die Zubereitung, die in dem erfindungsgemäß verwendeten Spender enthalten ist, in Form einer Emulsion, einer wässrigen Lösung oder als wasserfreie Formulierung vor.

Erfindungsgemäß vorteilhaft kann eine erfindungsgemäße Zubereitung neben einer oder mehreren Wasserphasen eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion) sein, wobei transparente oder transluzente Mikroemulsionen erfindungsgemäß besonders bevorzugt sind.

Erfindungsgemäß vorteilhaft kann die Zubereitung in Form einer Emulsion oder Dispersion vorliegen, wobei sowohl Makro- als auch Mikroemulsionen, O/W-Emulsionen, W/O-Emulsionen, S/W-Emulsionen, W/S-Emulsionen als auch multiple Emulsionen vorteilhaft im sinne der vorliegenden Erfindung eingesetzt werden können.

Die Zusammensetzungen enthalten gemäß der Erfindung außer den vorgenannten Substanzen gegebenenfalls weitere in der Kosmetik üblichen Zusatzstoffe, beispielsweise Parfum, Farbstoffe, antimikrobielle Stoffe, rückfettende Agentien, Komplexierungs- und Sequestrierungsagentien, Perlglanzagentien, Pflanzenextrakte, Selbstbräuner (z.B. DHA), Depigmentiermittel, Antischuppenwirkstoffe, Vitamine, weitere Wirkstoffe, Komplexe aus gamma-Oryzanol und Calciumsalzen, Niacinamid und dessen Derivate, Panthenol und dessen Derivate, Subtilisin, Mineralien, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate. Der pH-Wert der erfindungsgemäßen Zubereitungen wird dabei in der für den Fachmann üblichen Weise mit den entsprechenden Säuren (Milchsäure, Zitronensäure, Phosphorsäure etc.) und Basen (z.B. NaOH) eingestellt.

Erfindungsgemäß vorteilhaft weist die Zubereitung des erfindungsgemäßen Kosmetikums bzw. erfindungsgemäß verwendeten Spenders eine Viskosität von mindestens 500 mPas auf.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur: 25°C, Drehkörperdurchmesser 24 mm, Rotorgeschwindigkeit 62,5 U/min).

### II. Der erfindungsgemäße Spender

Zur Lösung der Aufgaben, welche der Erfindung zugrunde lagen, wird mit der vorliegenden Erfindung der eingangs genannte Spender (dies ist der in der EP-A-0 230 252 offenbarte Spender) dahingehend weitergebildet, dass die Fördereinrichtung ein zu dem Behälter und dem Kopfstück längsverschiebliches Förderelement umfasst, welches einen in der Förderkammer gleitverschieblichen Förderkolben aufweist, der mit einem Förderschaft verbunden ist, welcher einen Förderkanal umfänglich umgibt, der eine mit der Förderkammer kommunizierende Förderkanaleinlassöffnung und eine Förderkanalauslassöffnung aufweist, die durch eine Verschiebebewegung des Förderelementes relativ zu dem Kopfstück in eine Stellung bringbar ist, in der sich die Förderkanalauslassöffnung zu dem Ausgabekanal öffnet.

Bei dem erfindungsgemäßen Spender öffnet sich die Förderkammer zu dem Ausgabekanal über eine Förderkanalauslassöffnung, die über eine Längsverschiebung des Förderelementes relativ zu dem Kopfstück freigegeben wird. Diese Relativbewegung wird vorzugsweise dadurch erzielt, dass das Kopfstück handbetätigt wird, d. h. in axialer Richtung in Richtung auf den Behälter gleitverschoben wird. Der Durchlass des pastösen Produktes von der Förderkammer zu der Produktabgabeöffnung am Ende des Ausgabekanals wird dementsprechend bereits durch eine translatorische Bewegung des Kopfstückes relativ zu dem Förderelement freigegeben. Ein vorheriger Druckaufbau in der Förderkammer, wie er beim gattungsbildenden Stand der Technik zur Freigabe des Durchganges erforderlich war, ist nicht erforderlich. Dementsprechend werden die Betätigungskräfte zur Abgabe von pastösen Produkten aus dem Spender verringert.

Bei dem erfindungsgemäßen Spender ist nachgeordnet zu der Förderkammer ein Förderkanal vorgesehen, der von einem Förderschaft umgeben ist. Am Ende dieses Förderkanals wird das aus der Förderkammer ausgeförderte pastöse Produkt durch die Förderkanalauslassöffnung in den Ausgabekanal abgegeben. Erst nach Abgabe des Produktes aus der Förderkanalauslassöffnung steht dieses in dem Ausgabekanal an.

Der verbleibende Ausgabekanal ist jedenfalls kürzer als bei den üblicherweise verwendeten Spendern. Dementsprechend wird deutlich weniger Volumen pastöser Masse durch eventuelle Oxidationsvorgänge beeinträchtigt. Die verbleibende Restlänge des Ausgabekanals kann insbesondere bei solchen Produkten, die gegenüber Oxidation hochgradig anfällig sind, dadurch verkürzt werden, dass der Ausgabekanal sich in Verlängerung der Stirnseite des Kopfstückes nach außen öffnet.

Bei einer vorteilhaften Ausgestaltung des erfindungsgemäßen Spenders wird die Förderkanalauslassöffnung an der Umfangsfläche des Förderschaftes ausgespart und an dem Kopfstück eine die Förderkanalauslassöffnung in der Ausgangsstellung der Fördereinrichtung abdeckende Buchse vorgesehen, so dass sich bei einer Hubbewegung des Kopfteiles zum Ausfördern pastöser Masse auf einfache Weise eine Freigabe der Förderkanalauslassöffnung dadurch ergibt, dass der Förderschaft relativ zu der Buchse bewegt wird. Diese bevorzugte Ausgestaltung ist nicht nur einfach, sondern erlaubt auch eine Anordnung der Förderkanalauslassöffnung in unmittelbarer Nähe der Einlassöffnung des Ausgabekanals für das zu fördernde Produkt.

Mit Rücksicht auf eine gute axiale Führung der Fördereinrichtung relativ zu dem Kopfstück wird die vorerwähnte Buchse vorzugsweise als Führungsbuchse für die Fördereinrichtung ausgebildet und hat wenigstens eine mit der Umfangsfläche des Förderschaftes zusammenwirkende Führungsfläche.

Im Hinblick auf einen zwangsläufigen Verschluss der Förderkanalauslassöffnung bei Rückstellung des Kopfstückes in die Ausgangslage wird gemäß einer bevorzugten Weiterbildung der vorliegenden Erfindung vorgeschlagen, dass an dem Kopfteil und an der Fördereinrichtung Mitnehmermittel vorgesehen sind, durch welche die Fördereinrichtung nach Handbetätigung bei Rückstellung des Kopfteiles in die Ausgangsstellung mitgenommen wird.

Die vorerwähnten Mitnehmermittel sind auf einfache Weise vorzugsweise durch eine an der Buchse ausgebildete Mitnehmerschulter gebildet, die mit einem an dem Förderschaft angeformten Mitnehmerkranz zusammenwirkt. Dieser Mitnehmerkranz ist vorzugsweise endseitig an dem Förderschaft angeformt, so dass die unterhalb des Mitnehmerkranzes ausgesparte Förderkanalauslassöffnung in der Ausgangsstellung durch Anlage des Mitnehmerkranzes an Wandungen des Kopfteiles abgedichtet werden kann.

Bei der vorerwähnten bevorzugten Ausgestaltung kann das in dem Ausgabekanal anstehende Volumen dadurch weiter verringert werden, dass die Mitnehmerschulter endseitig an der Buchse und am Übergang zu dem Ausgabekanal und der Mitnehmerkranz im stirnseitigen Endbereich des endseitig verschlossenen Förderschaftes ausgebildet ist, wie dies gemäß einer bevorzugten Weiterbildung der vorliegenden Erfindung vorgeschlagen wird. Bei dieser bevorzugten Ausgestaltung deckt die endseitig an dem Förderschaft angeordnete Schaftkappe den Ausgabekanal in der Ausgangsstellung der Fördereinrichtung im wesentlichen bündig ab und weist vorzugsweise den Mitnehmerkranz auf.

Gemäß einer bevorzugten Weiterbildung der vorliegenden Erfindung erfolgt die Betätigung des Förderkolbens vorzugsweise über die endseitigen Stirnflächen der Führungsbuchse. Bei dieser bevorzugten Weiterbildung überragt der Förderkolben den Förderschaft radial zur Ausbildung einer ringförmigen Anlagefläche für eine Druckfläche, die stirnseitig an der Führungsbuchse ausgebildet ist und die in der Ausgangsstellung mit axialem Abstand zu der Anlagefläche angeordnet und durch axiales Verschieben des Kopfstückes in Richtung auf den Behälter an die Anlagefläche anlegbar ist.

Ebenfalls mit Rücksicht auf eine konstruktive Vereinfachung wird gemäß einer weiteren bevorzugten Ausgestaltung der vorliegenden Erfindung vorgeschlagen, die Innenwandung der Förderkammer durch eine Innenhülse auszubilden, welche an der kopfstückseitigen Stirnseite des Behälters vorgesehen ist. Dabei überragt die Innenhülse die Stirnseite des Behälters an der dem Kopfstück zugewandten Seite. Vorzugsweise ist die Innenhülse zur Verringerung der Bauteile einstückig an dem Behälter angeformt.

Zur einfachen Zentrierung des Kopfstückes bei der Montage des Spenders und leichten Befestigung des Kopfstückes an dem Behälter wird gemäß einer bevorzugten Weiterbildung der vorliegenden Erfindung ein Kopfgegenstück vorgeschlagen, das einen topfförmig auf die vorerwähnte Innenhülse gestülpten Haltezylinder sowie einen konzentrisch zu dem Haltezylinder angeordneten, die Gleitverschiebung des Kopfstückes führenden Führungszylinder aufweist. Der Führungszylinder und/oder der Haltezylinder erlauben eine leichte konzentrische Ausrichtung des Kopfteiles zu dem Zylinder. Ferner verbessert der Führungszylinder die Führung der Hubbewegung des Kopfstückes bei der Betätigung des Spenders.

Bei einer weiteren bevorzugten Ausgestaltung des erfindungsgemäßen Spenders, bei der das förderkammerseitige Ende des Führungszylinders einen Förderkolbenanschlag für den Förderkolben ausbildet, wird zum Einen eine relativ längliche Führung für den Förderkolben und zum Anderen auf einfache Weise eine Hubbegrenzung des Förderkolbens bereitgestellt. Eine derartige Hubbegrenzung hält beispielsweise das Kopfteil in der Ausgangslage an dem Behälter fest, wenn die Mitnehmermittel in Wirkverbindung stehen.

Vorzugsweise weist der Haltezylinder eine bodenseitige Ringschulter auf, welche eine Anlagefläche für eine Schraubenfeder ausbildet, die das Kopfstück in der Ausgangsstellung unter Vorspannung hält. Dies bietet den Vorteil, dass die äußere Umfangsfläche des Haltezylinders die Schraubenfeder innenseitig umgibt und somit ein Knicken der Feder verhindert. Die Ringschulter ist bei dieser bevorzugten Ausgestaltung auf die Stirnseite des Behälters aufgesetzt und ist somit insbesondere geeignet, das Kopfgegenstück in axialer Richtung gegenüber dem Behälter festzulegen.

Gemäß einer weiteren, besonders bevorzugten Ausgestaltung sind das Kopfgegenstück und das Kopfstück als vorgefertigte Spenderkomponente ausgebildet. Hierbei sind besonders bevorzugt das Kopfstück und das Kopfgegenstück mit ihrer äußeren Mantelfläche jeweils topfförmig übereinander geschoben, wobei das Kopfgegenstück wenigstens einen Anschlag zur Begrenzung der axialen Verschiebebewegung des Kopfstücks relativ zu dem Kopfgegenstück aufweist. In dem von den Mantelflächen umgebenden Innenraum befindet sich bei einer derartigen Ausgestaltung vorzugsweise ein Rückstellelement, beispielsweise die vorstehend erwähnte Schraubenfeder, welche das Kopfstück und das Kopfgegenstück in axialer Richtung beabstandet unter Vorspannung hält. Der vorerwähnte Anschlag grenzt die axiale Verschiebebewegung des Kopfstücks, d. h. sorgt nach dem Zusammenbau von Kopfstück und Kopfgegenstück unter Einschluss der Feder für den Zusammenhalt der beiden gegeneinander verschieblichen Bauteile. Die derart gebildete Spenderkomponente kann auf unterschiedlich ausgestaltete Behälter aufgesetzt werden, was eine wirtschaftliche Herstellung des Spenders für sehr unterschiedliche Anwendungen und Behältervolumina erlaubt.

Eine besonders einfache und haltbare Verbindung zwischen der vorgefertigten Spenderkomponente und dem Behälter wird dadurch gebildet, dass die Spenderkomponente mit dem Behälter über an dem Kopfgegenstück und der Stirnseite des Behälters ausgebildete Rastmittel verrastet sind.

Vorzugsweise ist bei dem erfindungsgemäßen Spender das Kopfstück derart längenverschieblich, dass das Kopfstück mittels Handbetätigung von der Ausgangsstellung zunächst um eine erste axiale Wegstrecke zur Anlage an den Förderkolben bei gleichzeitiger Freilegung der Förderkanalauslassöffnung in dem Ausgabekanal in eine Mittelposition bringbar ist und das Kopfteil danach bei fortschreitender axialer Verschiebung unter Mitnahme des Förderkolbens von der Mittelposition in eine Ausgabe-Endposition bringbar ist, in welcher die Förderkammer durch Verschiebung des Förderkolbens ihr kleinstes Volumen erreicht hat. Bei dieser bevorzugten Ausgestaltung erfolgt das Freilegen der Förderkanalauslassöffnung und das Komprimieren der Substanz in dem Förderkanal im Rahmen einer gleichgerichteten Bewegung des Kopfstücks in Richtung auf den Behälter. Diese bevorzugte Ausgestaltung erlaubt eine konstruktiv einfache Lösung des erfindungsgemäßen Spenders, bei welcher das Kopfstück unmittelbar auf den Förderkolben wirkt und diesen nach Freilegung der Förderkanalauslassöffnung zum Fördern pastöser Masse antreibt. Diese Bewegung des Kopfstücks erfolgt üblicherweise gegen die Kraft eines Vorspannelementes, beispielsweise einer Feder, die dafür Sorge trägt, dass bei einer Entlastung des Kopfstücks dieses von der Ausgabe-Endposition weg von dem Behälter drückt. Bei dieser Bewegung wird zuerst die axiale Wegstrecke a zurückgelegt, d. h. die Förderkanalauslassöffnung wird wieder verschlossen. Bei dieser Verschließbewegung findet eine Relativbewegung zwischen dem Förderschaft und dem Ausgabekanal statt, bei welcher das Volumen des Ausgabekanals an dessen Einlass vergrößert wird. Dadurch wird die in dem Ausgabekanal befindliche pastöse Masse in Richtung auf die Pumpkammer zurückgezogen, also von der Produktabgabeöffnung des Ausgabekanals in dem Kopfteil entfernt.

Gemäß der bevorzugten Ausgestaltung der Ansprüche 15 bis 18 befindet sich an dieser Produktabgabeöffnung ein Verschließteil. Das Verschließteil ist vorzugsweise derart beschaffen, dass es sich aufgrund einer Druckdifferenz zwischen dem Ausgabekanal und der Atmosphäre zur Abgabe des pastösen Produktes öffnet. Wird - wie vorstehend erwähnt - die pastöse Masse in dem Ausgabekanal weg von der Produktabgabeöffnung zurückgezogen, so führt dies zu einem relativen Unterdruck in dem Ausgabekanal, welcher dafür sorgt, dass das Verschließteil die Produktabgabeöffnung besonders wirkungsvoll abdichtet.

Im Hinblick auf eine möglichst gute Abdichtung ist es zu bevorzugen, die Produktabgabeöffnung um einen in dem Ausgabekanal angeordneten Verschlussdorn auszubilden.

Dieser Verschlussdorn ist vorzugsweise einstückig an dem Kopfteil ausgeformt. Das ebenfalls ringförmig ausgebildete Verschlussteil weist eine an dem Verschlussdorn dichtend anlegbare Dichtlippe auf, welche bei einem wirksamen Unterdruck den Ausgabekanal wirkungsvoll verschließt, jedoch beim Ausfördern des pastösen Produktes eine verhältnismäßig große Produktabgabeöffnung freigibt, durch welche bei relativ geringem Druckverlust das Produkt ausgefördert werden kann.

Besonders wirtschaftlich lässt sich ein Verschließteil hoher Wirksamkeit mittels Zweikomponenten-Spritzgiessen an dem Kopfteil ausformen, wie dies gemäß einer bevorzugten Weiterbildung der vorliegenden Erfindung vorgeschlagen wird. Bei dieser Ausgestaltung ist das Verschließteil fest mit dem Kopfteil verbunden. Vorzugsweise ist das Verschließteil aus einem weichelastischen Kunststoff, besonders bevorzugt aus einem thermoplastischen Elastomer gebildet. Es hat sich gezeigt, dass insbesondere durch einen thermoplastischen Elastomer eine wirkungsvolle Abdichtung der Produktabgabeöffnung erreicht werden kann.

Es hat sich gezeigt, dass das Material für das Dichtteil sich besonders bevorzugt zur Ausbildung einer Funktionsfläche an der stirnseitigen Außenfläche des Kopfteiles genutzt werden kann. Eine derartige Funktionsfläche kann beispielsweise eine die haptischen Eigenschaften verbessernde Drückerfläche sein, gegen welche der Benutzer des Spenders bei dessen Benutzung drückt. Eine derartige Funktionsfläche wird vorzugsweise durch einen Überzug zumindest stirnseitig an der Außenseite des Kopfteiles ausgebildet. Das Verschließteil sowie der Überzug sind einstückig ausgeformt, vorzugsweise mittels Zweikomponenten-Spritzgießen nach der spritzgießtechnischen Herstellung des Kopfteiles.

Weitere Einzelheiten, Vorteile und Merkmale der vorliegenden Erfindung ergeben sich aus der nachfolgenden Beschreibung eines Ausführungsbeispiels in Verbindung mit der Zeichnung. In dieser zeigen :
Figur 1 eine Längsschnittansicht eines ersten Ausführungsbeispieles eines erfindungsgemäßen Spenders ;
Figur 2 eine Längsschnittansicht eines zweiten Ausführungsbeispiels des erfindungsgemäßen Spenders.

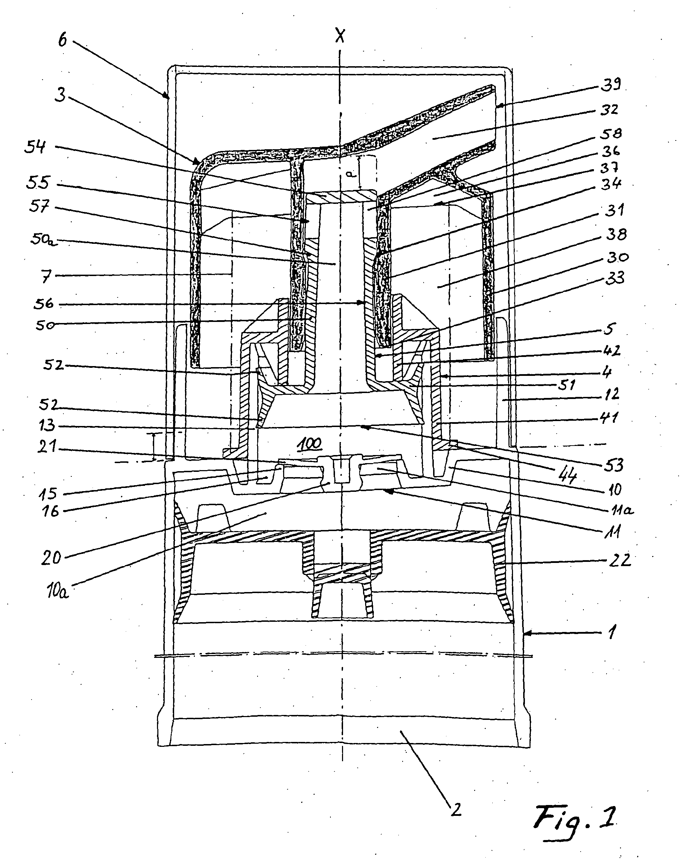

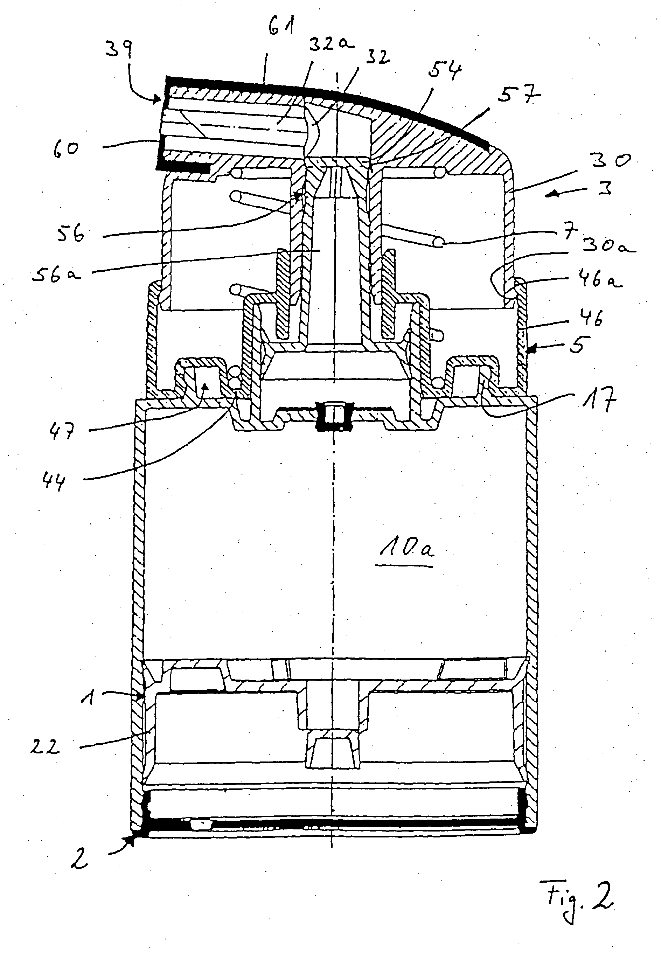

Das in Figur 1 gezeigte Ausführungsbeispiel eines erfindungsgemäßen Spenders hat einen Behälter 1, der topfförmig ausgebildet ist und an seiner Unterseite mit einer Bodenplatte 2 verbunden ist, welche mit dem Behälter 1 verrastet ist. An seiner anderen Stirnseite weist der Behälter 1 eine kopfseitige Abdeckung 10 auf, in der eine Behälteröffnung 11 ausgespart ist. Diese Abdeckung 10 ist auf der dem Behälter 1 abgewandten Seite zur Aufnahme eines Spenderkopfes bestehend aus einem Kopfstück 3, einem Kopfgegenstück 4 und einem Druckkolben 5 ausgebildet. Der Spender weist ferner eine auf eine sich oberhalb der Abdeckung 10 erstreckende Außenhülse 12 des Behälters 1 aufgeschobene Verschlusskappe 6 auf. Der Behälter 1, die Bodenplatte 2, das Kopfgegenstück 4 sowie der Druckkolben 5 sind als rotationssymmetrische Bauteile ausgebildet und zu einer Mittellängssachse X konzentrisch angeordnet. Zwischen dem Kopfstück 3 und dem Kopfgegenstück 4 befindet sich eine schematisch angedeutete Schraubenfeder 7, durch welche das Kopfstück 3 in der in Figur 1 gezeigten Ausgangsstellung gegenüber dem Kopfgegenstück 4 vorgespannt gehalten ist.

Das Kopfstück 3 weist einen zylindrischen Außenmantel 30 auf, welcher radial innerhalb unmittelbar benachbart zu der Außenhülse 12 des Behälters 1 und konzentrisch zu dieser angeordnet ist. Die Außenhülse 12 des Behälters 1 überragt das behälterseitige Ende des Außenmantels 30 in axialer Richtung. Dementsprechend erscheint das in Figur 1 gezeigte Ausführungsbeispiel des Spenders auch bei abgenommener Verschlusskappe als geschlossene Einheit bestehend aus dem Behälter 1 und dem Kopfstück 3. Wie nachfolgend näher erläutert wird, ist das Kopfstück 3 sowie der Druckkolben 5 längsverschieblich gegenüber dem Behälter 1 gehalten, wobei der Druckkolben 5 darüber hinaus längsverschieblich gegenüber dem Kopfstück 3 ist.

Die zylindrische Wandung des Behälters 1 umschließt einen Innenraum 10a zur Aufnahme der kosmetischen oder dermatologischen Zubereitung. In der Behälteröffnung 11 erstrecken sich sternförmig ausgerichtete Haltestege 11 a. Auf der dem Innenraum 10a abgewandten Seite der Abdeckung 10 ist konzentrisch zu der Behälteröffnung 11 eine zylindrische Innenhülse 13 angeordnet, die in axialer Richtung von der Außenhülse 12 überragt wird und die eine Förderkammer 100 umgibt. Die Innenwandung der Innenhülse 13 ist glatt. Der Boden der Förderkammer 100 wird durch die Abdeckung 10 des Behälters 1 gebildet. Die Abdeckung 10 weist einen in die Förderkammer 100 hineinragenden Ringkranz 15 auf, welcher die Behälteröffnung 11 umgibt und zwischen sich und der Innenhülse 13 einen Ringspalt 16 bildet.

Der Druckkolben 5 weist einen im wesentlichen zylindrischen, innen hohlen Förderschaft 50 auf, an dessen einem Ende ein Förderkolben 51 einstückig angeformt ist. Der Förderkolben 51 überragt den Förderschaft 50 radial und hat an seiner äußeren Umfangsfläche jeweils obere und untere Dichtlippen 52, die den im wesentlichen ringförmig ausgebildeten Förderkolben 51 in axialer Richtung überragen. Auf einer dem Förderschaft 50 zugewandten Stirnseite bildet der Förderkolben 51 eine ringförmige Anlagefläche aus.

Der Förderschaft 50 weist an seinem einen Ende eine in der Mitte des ringförmigen Förderkolbens 51 ausgesparte Förderkanaleinlassöffnung 53 auf. An seinem anderen Ende ist der Förderschaft 50 stirnseitig durch eine Schaftkappe 54 verschlossen. Die Schaftkappe 54 deckt einen Zylinderabschnitt 55 des Förderschaftes 50 ab, der gegenüber dem übrigen Schaftbereich 56 im Durchmesser vergrößert ist. Zwischen diesem Schaftbereich 56 und dem Zylinderabschnitt 55 befindet sich ein schräg nach außen geneigter Mitnehmerkranz 57. Zwischen dem Mitnehmerkranz 57 und der Schaftkappe 54 sind an der äusseren Umfangsfläche des Zylinderabschnitts 55 mehrere Förderkanalauslassöffnungen 58 verteilt ausgespart. Zwischen den Förderkanalauslassöffnungen 58 erstrecken sich in Umfangsrichtung Haltestege, welche die Schaftkappe 54 tragen. Die Förderkanaleinlassöffnung 53 kommuniziert über einen von dem Förderschaft 50 umgebenden Förderkanal 50a mit den Förderkanalauslassöffnungen 58 und bildet eine Förderpassage für die pastöse Substanz, die frei von Rückschlagventilen ist.

Das Kopfstück 3 weist einen, zylindrischen Außenmantel 30 auf, zu dem konzentrisch eine innen hohle Führungsbuchse 31 angeordnet ist, die mit einem Ausgabekanal 32 kommuniziert. Das stirnseitige Ende der Führungsbuchse 32 bildet eine stirnseitige Druckfläche 33 aus, welche in axialer Richtung von dem Außenmantel 30 überragt wird. Die Führungsbuchse 31 weist benachbart zu der stirnseitigen Druckfläche 33 einen ersten Buchsenabschnitt auf, der einen geringeren Innendurchmesser hat, als der in aus Förderrichtung der pastösen Substanz dahinter liegender zweiter Buchsenabschnitt. Zwischen dem ersten und dem zweiten Buchsenabschnitt ist eine Mitnehmerschulter 34 ausgebildet, welche die beiden Abschnitte unterschiedlicher Buchsendurchmesser über eine Schräge miteinander verbindet. Der zweite Buchsenabschnitt mündet in einen in den von der Mittellängsachse X seitlich abgehenden Ausgabekanal 32.

In etwa rechtwinkliger Erstreckung zu der Mittellängsachse X weist das Kopfstück 3 an Rippen 36 ausgebildete Federanlageflächen 37 auf. Die Rippen 36 erstrecken sich in etwa sternförmig von der Buchse 31 zu der Innenfläche des Außenmantels 30. Dementsprechend wird zwischen der Innenfläche des Außenmantels 30, der Außenfläche der Führungsbuchse 31 und dem Federanlageflächen 37 ein zu der Unterseite des Kopfstückes 3 offener Ringraum 38 ausgebildet.

Das Kopfteil 3 ist zu der behälterwärtigen Seite des Außenmantels 30 offen und oberhalb dieser Stirnseite im wesentlichen nach Art einer Kappe ausgebildet. An der der Stirnseite des Außenmantels 30 abgewandten Oberseite des Kopfteiles 3 befindet sich eine Produktabgabeöffnung 39 des Ausgabekanals 32.

Das Kopfgegenstück 4 weist im wesentlichen zwei konzentrische Zylinderabschnitte aufweist, nämlich einen äusseren Haltezylinder 41 und einen im Durchmesser kleineren Führungszylinder 42. Der Haltezylinder 41 überragt den Führungszylinder 42 auf der dem Behälter 1 zugewandten Seite, wohingegen der Führungszylinder 42 den Haltezylinder 41 auf der anderen Seite überragt. An der dem Behälter 1 abgewandten Stirnseite des Haltezylinders 41 ist ein radial von dort nach innen sich erstreckender Ringsteg vorgesehen, der etwa mittig an die Außenfläche des Führungszylinders 42 stößt.

Der Haltezylinder 41 weist an seiner behälterseitigen Stirnseite eine nach außen vorspringende umlaufende Ringschulter auf. Die behälterseitige Stirnseite des Führungszylinders 42 bildet einen Förderkolbenschlag aus.

Im zusammengebauten Zustand befindet sich der Förderkolben 51 des Druckkolbens 5 gleitverschieblich in der Innenhülse 13 des Behälters 1 und deckt somit stirnseitig die Förderkammer 100 ab. Das Kopfgegenstück 4 ist konzentrisch zu der Innenhülse 13 angeordnet und mit seinem Haltezylinder 41 topfförmig über die Innenhülse 13 geschoben. Die Ringschulter des Kopfgegenstücks 4 liegt an der dem Behälter 1 abgewandten Stirnseite der Abdeckung 10 an.

Die Ringschulter des Kopfgegenstückes 4 befindet sich etwa im Bereich des stirnseitigen Endes der Innenhülse 13. Der sich hieran radial nach innen anschließende Führungszylinder 42 umgibt Ende der Führungsbuchse 31 des Kopfteils 3. Radial innerhalb dieser Führungsbuchse 31 befindet sich der Förderschaft 50 mit seinem Schaftbereich 56 kleineren Durchmessers. Der Förderkolben 51 des Druckkolbens 5 ist gleitverschieblich an der Innenwandung der Innenhülse 13 angeordnet. Die ringförmige Anlagefläche des Förderkolbens 51 liegt stirnseitig an dem Förderkolbenanschlag des Führungszylinders 42 an. Hierdurch wird die von der Feder 7 auf das Kopfstück 3 ausgeübte Vorspannkraft gehalten, welche über die Anlage von Mitnehmerschulter 34 und Mitnehmerkranz 57 den Druckkolben 5 in einer Richtung weg von dem Behälter 1 vorspannt.

Zwischen der Förderkammer 100 und dem Innenraum 12 des Behälters 1 befindet sich ein in bekannter Weise ausgebildetes Behälterventil 20, das mit seiner Dichtungsscheibe 21 gegen den Ringkranz 15 der Abdeckung 10 anliegt und den Innenraum 10a gegenüber der Förderkammer 100 abdichtet.

Im nicht benutzen Zustand befindet sich der Spender in Ausgangsstellung (0).

Bei der Benutzung des Spenders drückt ein Benutzer das Kopfstück 3 in Richtung auf den Behälter 1. Aufgrund der Inkompressibilität des in der Förderkammer 100 und dem Förderkanal 50a enthaltenen Substanz verharrt der Druckkolben 5 in seiner Lage. Das Kopfstück 3 bewegt sich relativ zu dem Druckkolben 5 in Richtung auf den Behälter 1 zu. Die formschlüssige Anlage zwischen dem Mitnehmerkranz 57 und der Mitnehmerschulter 34 wird gelöst, bis die Schaftkappe 54 gegen die Innenfläche des Buchsenkopfes 35 stößt bzw. - je nach Ausgestaltung - die stirnseitige Druckfläche 33 am Ende der Führungsbuchse 31 an der ringförmigen Anlagefläche 51a des Förderkolbens 51 zur Anlage (Mittelposition M) kommt. Nach dieser axialen Verschiebung um den Verschiebeweg a liegen die Förderkanalauslassöffnungen 53 in dem Ausgabekanal 32 frei.

Das Kopfstück 3 wird bei dieser wie auch bei jeder übrigen axialen Relativbewegung zwischen dem Kopfstück 3 und dem Kopfgegenstück 4 bzw. zwischen dem Kopfstück 3 und dem Behälter 1 durch die Anlage der Außenumfangsfläche der Führungsbuchse 31 an der Innenumfangsfläche des Führungszylinders 42 gleitend geführt. Die Relativbewegung zwischen dem Kopfstück 3 und dem Druckkolben 5 wird über die Anlage der Umfangsfläche des zweiten Schaftabschnittes an dem Schaftbereich 56 geführt.

Bei fortschreitender Druckbewegung des Kopfstückes 3 in Richtung auf den Behälter 1 wird der Druckkolben 5 mitgenommen. Hierbei verringert sich das Volumen der Förderkammer 100, so dass das sich in Förderrichtung hinter dem Behälterventil 30 befindende pastöse Produkt über die Förderkanalauslassöffnung 53 in den Ausgabekanal 32 abgegeben wird. Das pastöse Produkt verlässt den Ausgabekanal über dessen Produktabgabeöffnung 39.

Am Ende dieser Relativbewegung des Kopfstückes 3 in Richtung auf den Behälter 1 stoßen die behälterseitigen Dichtlippen 52 des Druckkolbens 5 gegen die Stirnseite des Ringspalts 16. In dieser Ausgabe-Endposition V hat die Förderkammer 100 ihr kleinstes Volumen erreicht.

Wird nunmehr das Kopfstück 3 von dem Benutzer freigegeben, so drückt die Schraubenfeder 7 das Kopfstück 3 in entgegen gesetzter Richtung zurück. Hierbei verharrt zunächst der Druckkolben 5 in seiner Ausgabe-Endposition V. Lediglich das Kopfstück 3 bewegt sich weg von dem Behälter 1, und zwar so lange, bis die Mitnehmerschulter 34 zur Anlage an den Mitnehmerkranz 57 kommt.

Bei dieser axialen Verschiebung um die Wegstrecke a wird das in dem Ausgabekanal 32 befindliche pastöse Produkt in den hierbei gebildeten Raum zwischen der Schaftkappe 54 und der Innenseite des Buchsenkopfes 35 zurückgezogen. Das pastöse Produkt liegt danach am Ende dieser Verschiebewegung nicht mehr unmittelbar an der Produktausgabeöffnung 39 des Ausgabekanals 32 an, wodurch verhindert wird, dass pastöses Produkt am Ende des Fördervorgangs aus dem Ausgabekanal 32 tropft bzw. durch Verschmutzung im Bereich der Produktausgabeöffnung 39 beeinträchtigt wird.

Nach der Verlagerung um die Wegstrecke a und der Anlage von Mitnehmerkranz 57 und Mitnehmerschulter 34 wird auch der Druckkolben 5 bei fortschreitender Bewegung des Kopfstücks 3 in Richtung auf die Ausgangsstellung zurückbewegt, die dann erreicht wird, wenn der Förderkolbenanschlag gegen die ringförmige Anlagefläche des Förderkolbens 51 anliegt. Bei der Relativbewegung des Druckkolbens 5 weg von dem Behälter 1 wird pastöses Produkt aus dem Innenraum 10a des Behälters 1 durch die Behälteröffnung 11 in die Förderkammer 100 gefördert. Der hierbei in dem Innenraum 10a entstehende relative Unterdruck führt in an sich bekannter Weise zu einer Nachlaufbewegung des in dem Innenraum 12 befindlichen Nachlaufkolbens 22.

In Fig. 2 ist ein zweites Ausführungsbeispiel des erfindungsgemäßen Spenders gezeigt.

Gleiche Teile sind bei diesem Ausführungsbeispiel gegenüber dem vorher Diskutierten mit gleichen Bezugszeichnen gekennzeichnet. Der Behälter 1 des in Fig. 7 gezeigten Ausführungsbeispiels ist im Wesentlichen identisch wie der vorher beschriebene Behäi- ter ausgebildet mit einer Behälteraußenwand, die einen Innenraum 10a umgibt, in dem ein Nachlaufkolben 22 längsverschieblich angeordnet ist und welcher von einer Bodenplatte 2 verschlossen ist. Im Gegensatz zu dem vorher beschriebenen Ausführungsbeispiel weist der Behälter 1 an seiner stirnseitigen Abdeckung einen umlaufenden Rastring 17 auf. Das Kopfgegenstück 4 ist über die Ringschulter 44 radial nach außen verlängert und hat eine sich im Wesentlichen parallel zu dem Haltezylinder 51 erstreckende zylinderförmige Außenwand 46, deren Durchmesser größer als der Durchmesser des Außenmantels 30 des Kopfstücks 3 ist. Zwischen der Außenwand 46 und dem Haltezylinder 41 ist an der behälterwärtigen Unterseite des Kopfgegenstücks 4 eine Rastausnehmung 47 ausgeformt, welche mit dem Rastring 17 zur Ausbildung einer Rastverbindung zwischen dem Kopfstück 4 und dem Behälter 1 zusammenwirkt.

Bei dem in Fig. 2 gezeigten Ausführungsbeispiel ist das Kopfgegenstück 4 zusammen mit dem Kopfstück 3 als vorgefertigte Spenderkomponente ausgebildet. Das dem Behälter 1 abgewandte freie Ende der Außenwand 46 des Kopfgegenstücks 4 ist radial zur Ausbildung einer Rastnase 46a nach innen abgekröpft und überragt in axialer Richtung einen Ringwulst 30a, der an der Außenseite des Außenmantels 30 an dem Kopfstück 3 vorgesehen ist. Hierdurch ist ein Anschlag gebildet, durch welchen das Kopfgegenstück 4 unverlierbar mit dem Kopfstück 3 verbunden ist. Dieser Anschlag hält die von der Feder 7 aufgebrachten Federkräfte. Die das Kopfstück 3 und das Kopfgegenstück 4 umfassende Spenderkomponente kann somit vor der Montage auf den Behälter 1 vormontiert werden. Hierzu wird die Feder 7 in den Hohlraum zwischen dem Kopfstück 3 und dem Kopfgegenstück 4 eingesetzt. Die beiden Bauteile 3,4 werden so weit in axialer Richtung ineinander geschoben, bis der Ringwulst 30a an dem nach innen umbogenen Ende der Außenwand 46 vorbei geglitten ist.

Bei dem in Fig. 2 gezeigten Ausführungsbeispiel weist der Druckkolben 5 einen Mitnehmerkranz 57 auf, der an der Schaftkappe 54 ausgeformt ist. Dementsprechend dichtet der Mitnehmerkranz 57 bei der in Fig. 2 gezeigten Ausgangsstellung den Ausgabekanal 32 ab. Der Förderschaft 50 hat einen Schaftbereich 56 mit verkleinertem Durchmesser, dessen Längserstreckung der axialen Wegstrecke a entspricht. Dementsprechend wird die axiale Verschieblichkeit des Druckkolbens 5 gegenüber dem Kopfteil 3 durch die Schaftkappe 54 einerseits und die Längserstreckung des Schaftbereiches 56 mit verringertem Durchmesser andererseits festgelegt.

Bei dem in Fig. 2 gezeigten Ausführungsbeispiel besteht gegenüber dem vorerwähnten ersten Ausführungsbeispiel der weitere Unterschied, dass in dem Ausgabekanal 32 ein Verschlussdorn 32a vorgesehen ist, welcher einstückig an dem Kopfstück 3 ausgeformt ist. Durch den Verschlussdorn 32a wird die Produktabgabeöffnung 39 ringförmig. Die Produktabgabeöffnung 39 ist bei dem gezeigten Ausführungsbeispiel durch ein ringförmiges Verschließteil 60 abgedeckt, welches als separates Bauteil aus einem thermoplastischen Elastomer mit dem Kopfstück 3 verbunden ist. Das Verschließteil 60 liegt in der in Fig. 7 Ausgangsstellung an der äußeren Umfangsfläche und an Teilen der Stirnseite, insbesondere aber der Umfangsfläche des Verschlussdorns 32a an und dichtet somit den Ausgabekanal 32 ab. Einstückig mit dem Verschließteil 60 ist ein Überzug 61, der stoffidentisch wie das Verschließteil 60 ausgebildet ist und welcher sich über einen überwiegenden Teil der stirnseitigen Abdeckung des Kopfstücks 3 erstreckt. Durch diesen Überzug 61 wird eine rutschfeste Funktionsfläche an dem Kopfstück 3 ausgebildet.

Bei der Betätigung des in Fig. 2 gezeigten Spenders laufen die vorstehend insbesondere unter Bezugnahme auf die Fig. 1 erläuterten Vorgänge ab. Es besteht jedoch gegenüber dem vorerwähnten Ausführungsbeispiel vorliegend der Unterschied, dass beim Zurückstellen von Druckkolben 5 und Kopfstück 3 der Ausgabekanal gegenüber der Umgebung abgedichtet wird. Bei einer Relativbewegung des Druckkolbens 5 relativ zu dem Kopfstück 3 in Richtung auf den Behälter 1 zu, wird das in dem Ausgabekanal 32 befindliche Produkt - wie vorsteht bereits erwähnt - entgegen der Förderrichtung zurück in das Innere des Kopfstücks 3 gezogen. Bei dem in Fig. 2 dargestellten Ausführungsbeispiel bewirkt der hierbei entstehende Druckgradient zwischen der Atmosphäre und dem Ausgabekanal 32 ein vollständig dichtendes Anliegen des Verschließteiles 60 an den Flächen des Verschlussdornes 32a. Dementsprechend bleibt in dem Ausgabekanal 32 anliegendes pastöses Produkt nahezu unbeeinflusst von eventuellen Oxidationsvorgängen. Zusätzlich dichtet die Schaftkappe 54 den Förderkanal 50a gegenüber dem Ausgabekanal 32 ab, so dass insbesondere eine Beeinträchtigung in dem Förderkanal 50a befindlichen pastösen Produktes durch eventuell in den Ausgabekanal 32 eindringende Luft in jedem Fall vermieden wird.

Die beiden vorstehend beschriebenen Ausführungsbeispiele weisen gemeinsam den Vorteil auf, dass die Förderkanalöffnungen 58 erst nach einer Relativbewegung zwischen dem Kopfteil 3 und dem Druckkolben 5 in dem Ausgabekanal 32 freiliegen. Zum Fördern des pastösen Produktes aus der Förderkammer in Richtung auf die Produktabgabeöffnung 32a ist es nicht erforderlich, dass der zunächst aufgebaute Innendruck in der Förderkammer 100 dazu genutzt wird, ein in Förderrichtung dahinter liegendes Rückschlagventil zu öffnen. Dementsprechend kann das pastöse Produkt mit geringerem Kraftaufwand ausgefördert werden. Weiterhin bieten beide vorerwähnten Ausführungsbeispiele den Vorteil, dass das pastöse Produkt entgegen der Förderrichtung nach der Betätigung des Kopfstückes in dem Ausgabekanal 32 zurückgezogen wird, wobei das in Fig. 2 gezeigte Ausführungsbeispiel den zulässigen Vorteil hat, dass durch die dichtende Anlage des Verschließteiles 60 an dem Verschlussdorn 32a das in dem Spender enthaltene pastöse Produkt sicher vor einer Beeinträchtigung beispielsweise durch Sauerstoff in der Luft geschützt wird.

### Bezugszeichenliste

- 1: Behälter
- 2: Bodenplatte
- 3: Kopfstück
- 4: Kopfgegenstück
- 5: Druckkolben
- 6: Verschlusskappe
- 7: Schraubenfeder
- 10: Abdeckung
- 10a: Innenraum
- 11: Behälteröffnung
- 11a: Haltesteg
- 12: Außenhülse
- 13: Innenhülse
- 15: Ringkranz
- 16: Ringspalt
- 17: Rastring
- 20: Behälterventil
- 21: Ventilscheibe
- 22: Nachlaufkolben
- 30: Außenmantel
- 30a: Ringwulst
- 31: Führungsbuchse
- 32: Ausgabekanal
- 32a: Verschlussdorn
- 33: Druckfläche
- 34: Mitnehmerschulter
- 36: Rippe
- 37: Federanlagefläche
- 38: Ringraum
- 39: Produktabgabeöffnung
- 41: Haltezylinder
- 42: Führungszylinder
- 44: Ringschulter
- 46: Außenwand
- 46a: Rastnase
- 47: Rastausnehmung
- 50: Förderschaft
- 50a: Förderkanal
- 51: Förderkolben
- 52: Dichtlippen
- 53: Förderkanaleinlassöffnung
- 54: Schaftkappe
- 55: Zylinderabschnitt
- 56: Schaftbereich
- 57: Mitnehmerkranz
- 58: Förderkanalauslassöffnung
- 60: Verschiiessteil
- 61: Überzug
- 100: Förderkammer

Erfindungsgemäß ist die Verwendung des erfindungsgemäßen Kosmetikums bzw. des erfindungsgemäßen Spenders zur Reinigung und Pflege der Haut und/oder Hautanhangsgebilde. Als Hautanhangsgebilde werden dabei insbesondere die Haare und Nägel verstanden.

Erfindungsgemäß ist die Verwendung eines Spenders für pastöse Produkte mit ein eine kosmetische und/oder dermatologische Zubereitung enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet dass die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet,
zur Aufbewahrung und Darreichung kosmetischer und/oder dermatologischer Zubereitungen wie sie im Rahmen dieser Offenbarung beschrieben wurden.

Insbesondere ist die Verwendung der Zubereitung des erfindungsgemäßen Kosmetikums bzw. des erfindungsgemäßen Spenders als Duschgel, Wannenbad oder Haarwaschmittel (Haarshampoo) erfindungsgemäß.

Erfindungsgemäß ist die Verwendung der Zubereitung des erfindungsgemäßen Kosmetikums bzw. des erfindungsgemäßen Spenders als Hautcreme, Lotion oder Gel zur Pflege der Haut erfindungsgemäß vorteilhaft. Dabei ist es erfindungsgemäß bevorzugt, wenn es sich bei der kosmetischen und/oder dermatologischen Zubereitung um eine Zubereitung zum Schutz der Haut vor Sonnenlicht (Sonnenschutzmittel) oder zur Prophylaxe, Behandlung und oder Pflege von trockender Haut, unreiner bis fettiger Haut, Akne, Altershaut, Hautalterung, Babyhaut und/oder Neurodermitis handelt.

Ferner eignen sich die erfindungsgemäßen Produkte hervorragend zur Reinigung und Pflege von Gegenständen des täglichen Lebens (z.B. Geschirr, Tisch- und Schrankflächen, Autos, Kleidungsstücke, Wäsche).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispielrezepturen für die kosmetische und/oder dermatoloctische Zubereitung

### O/W-Emulsionen

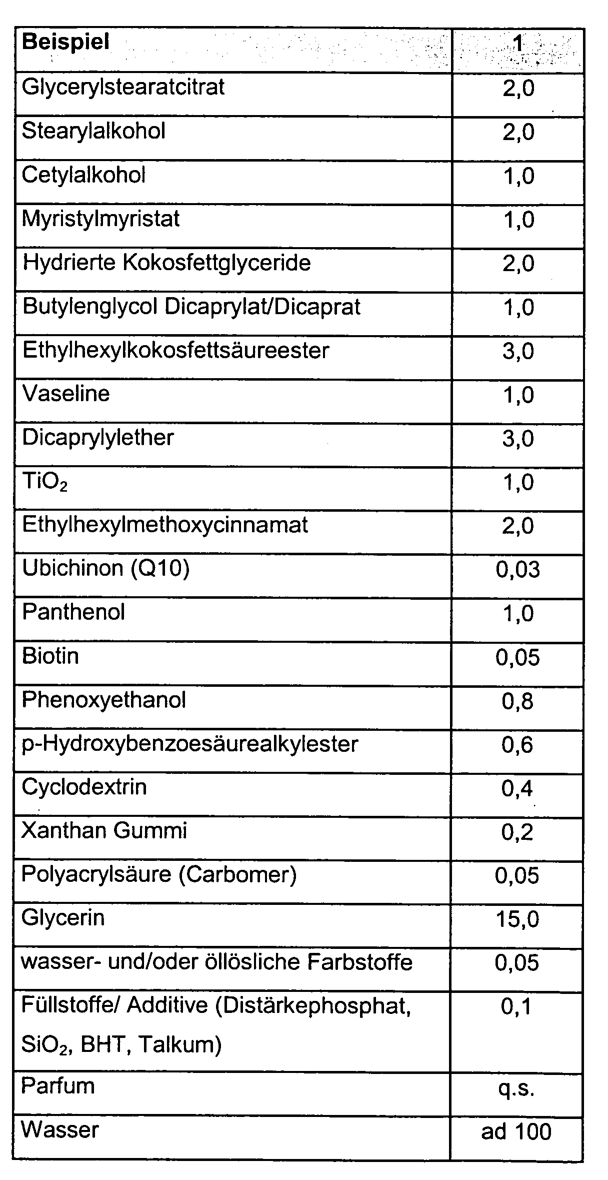

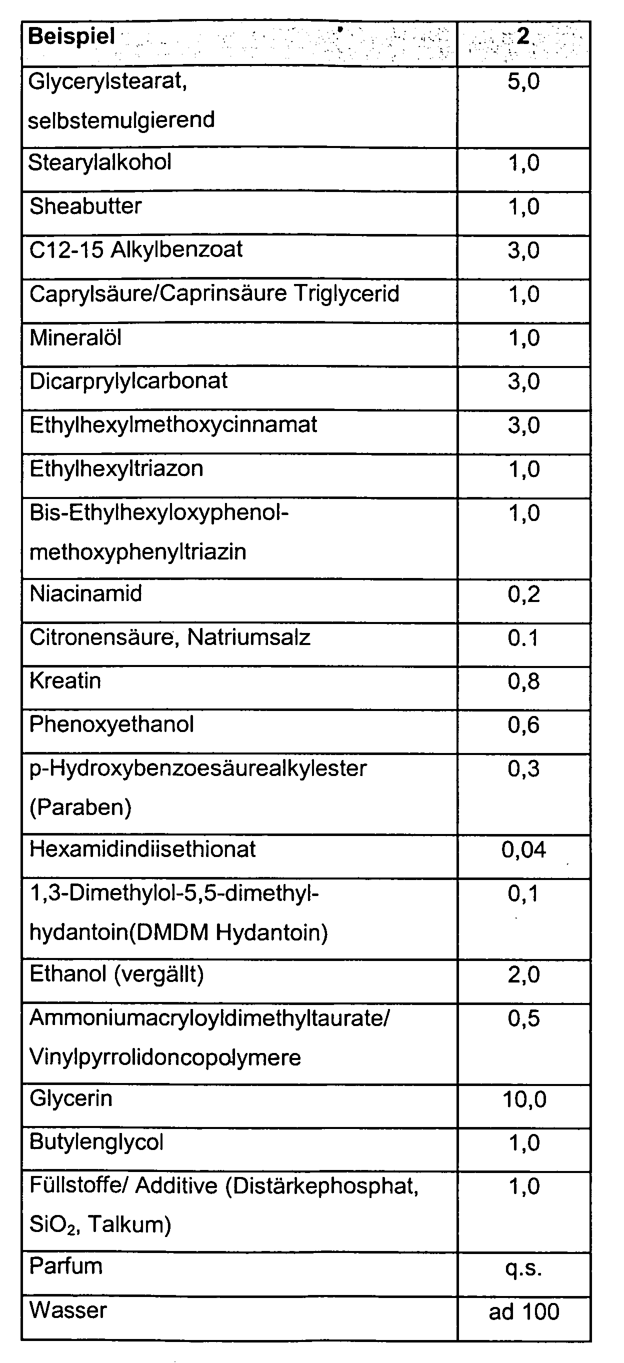

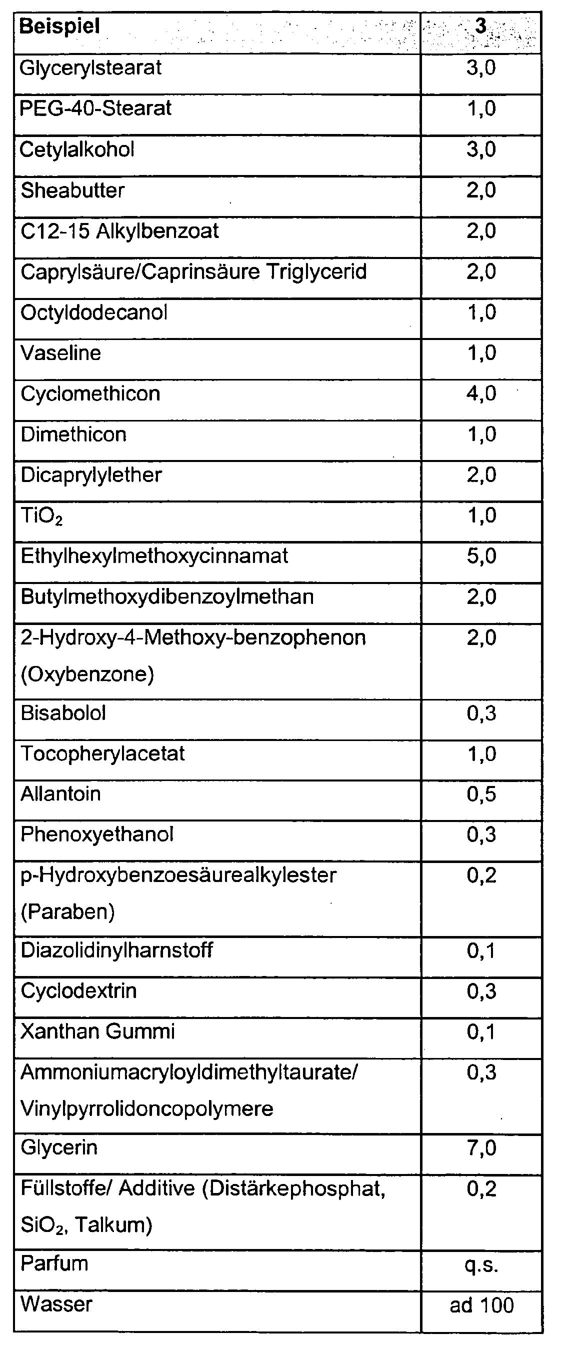

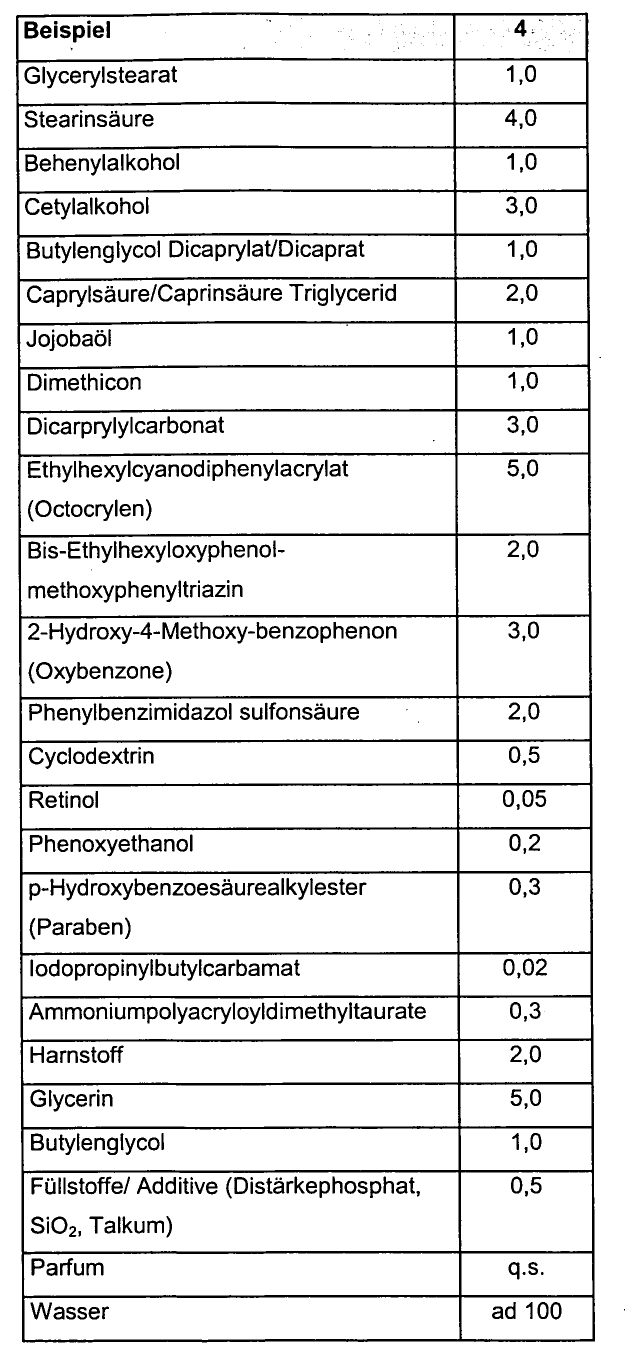

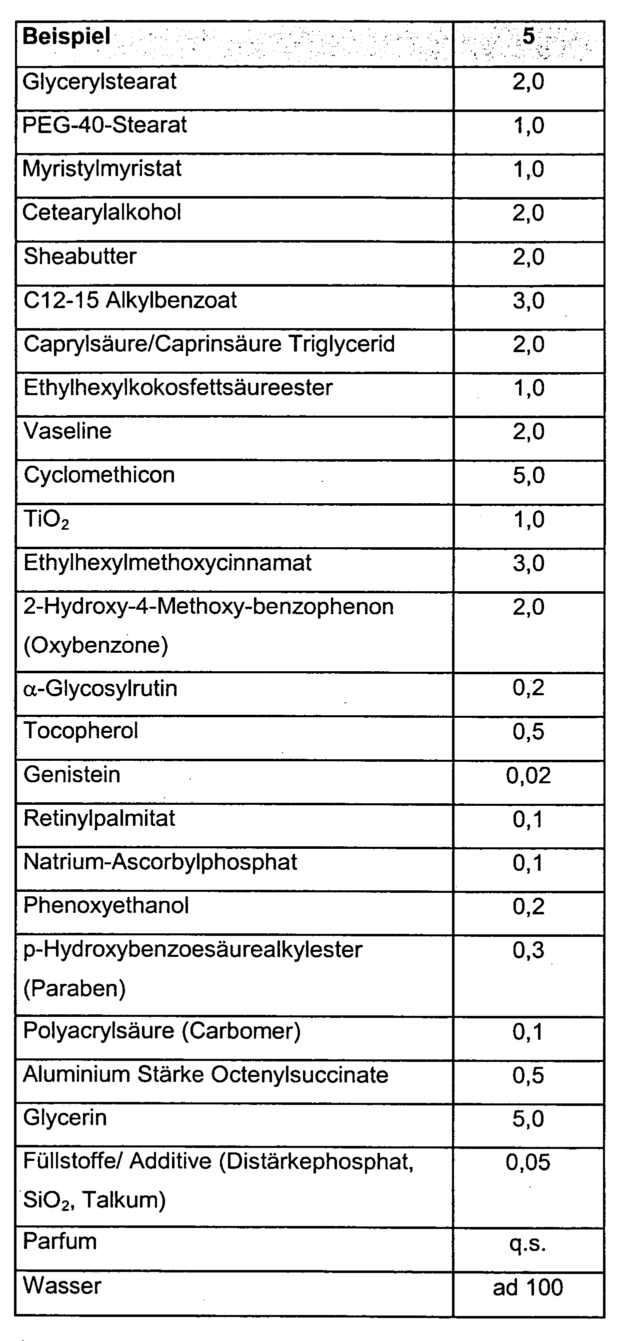

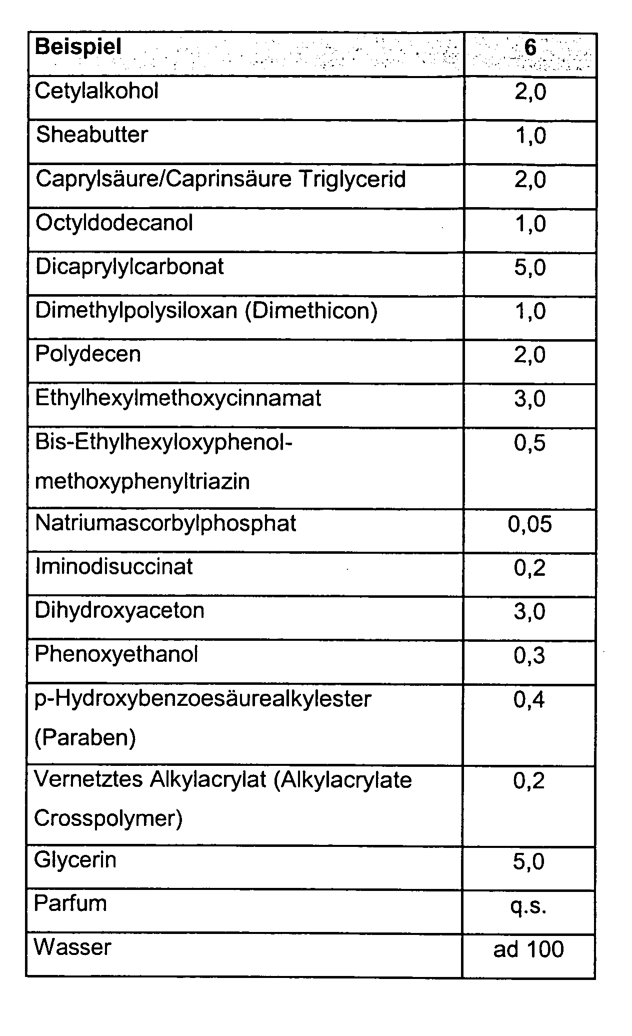

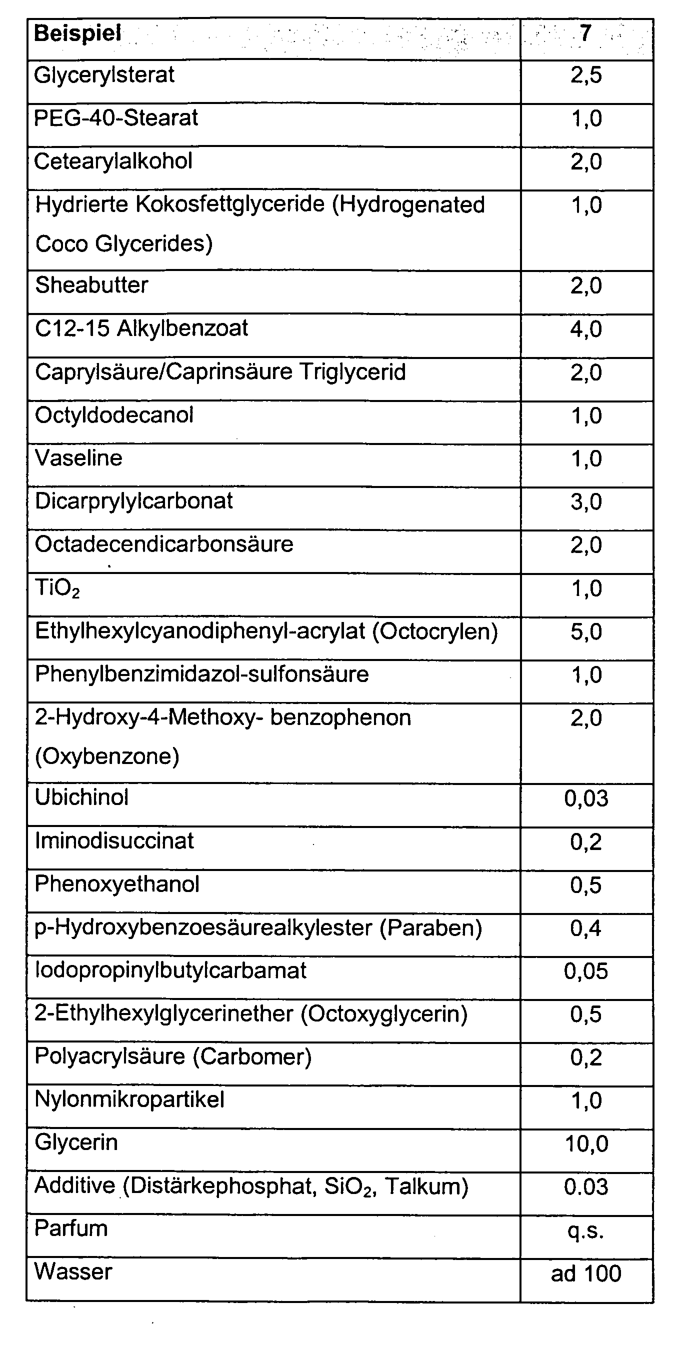

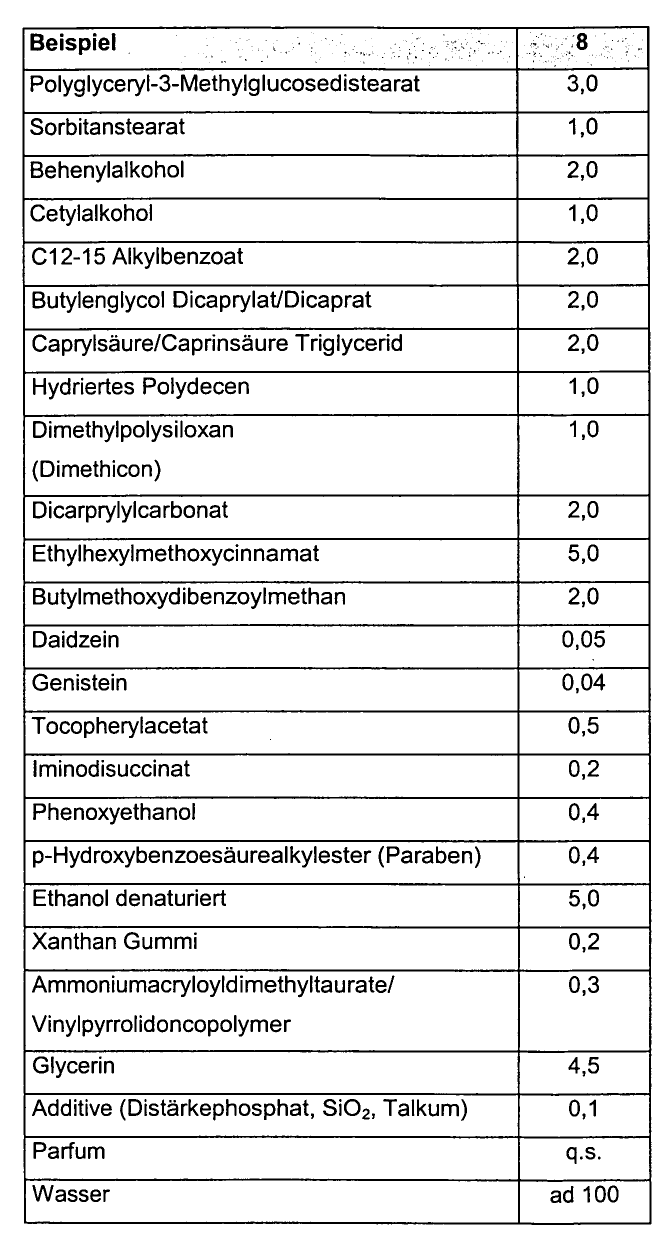

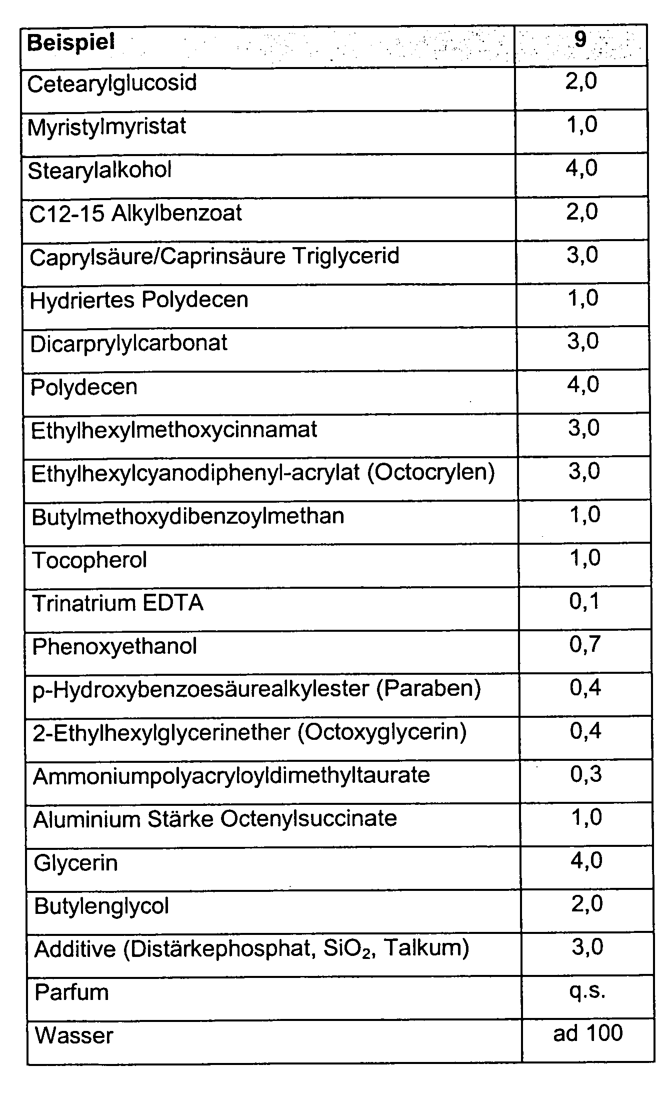

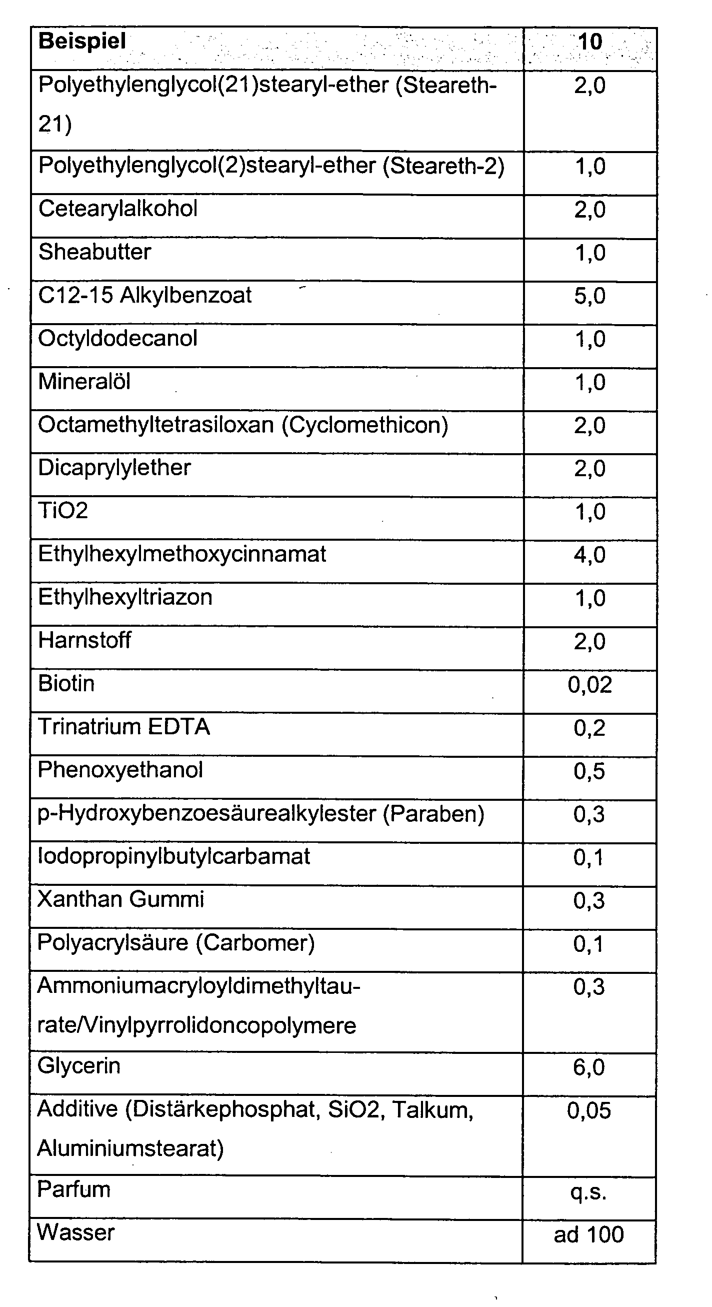

### W/O-Emulsionen

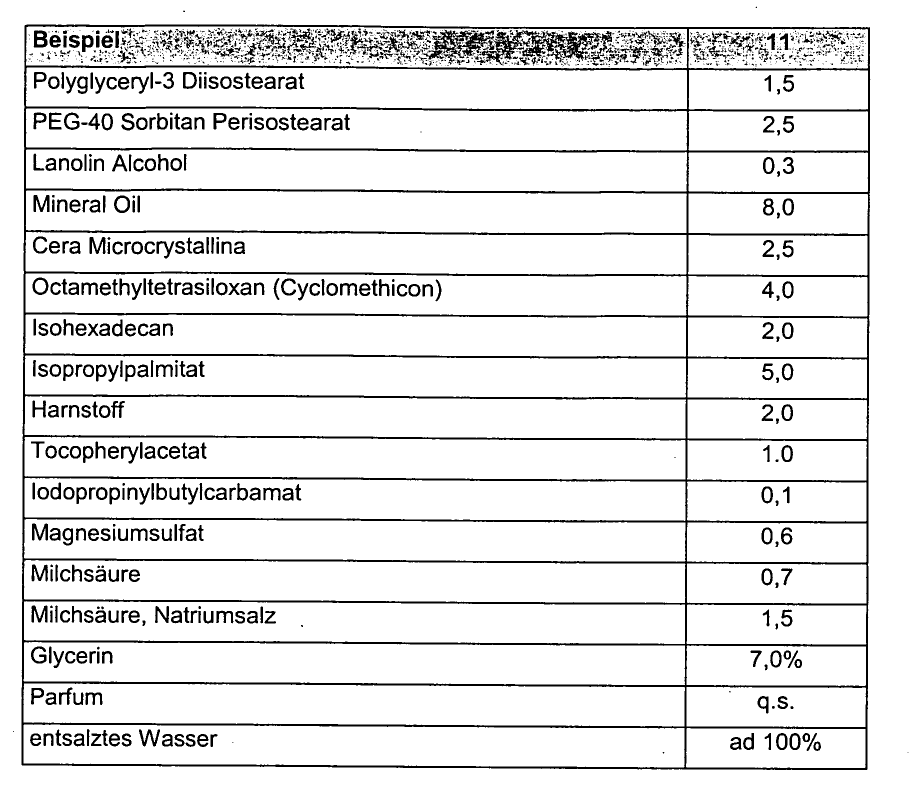

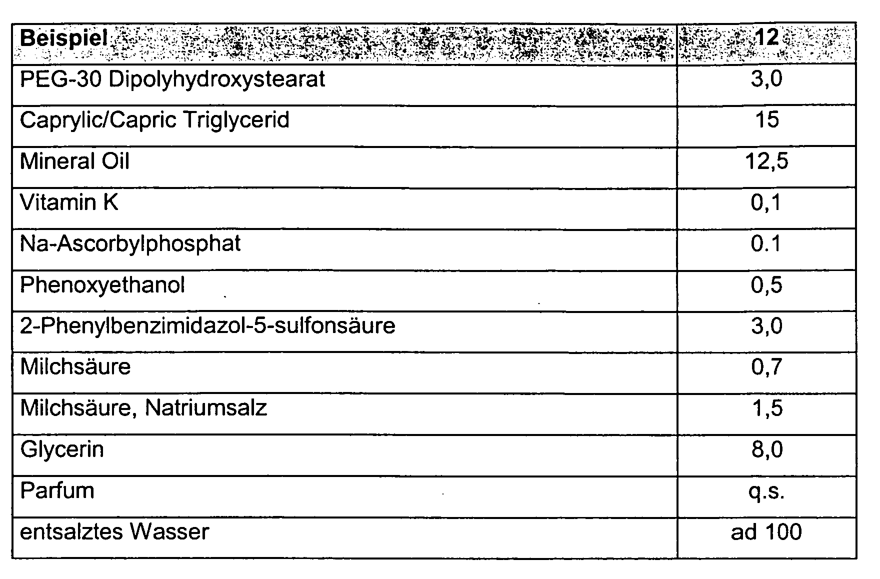

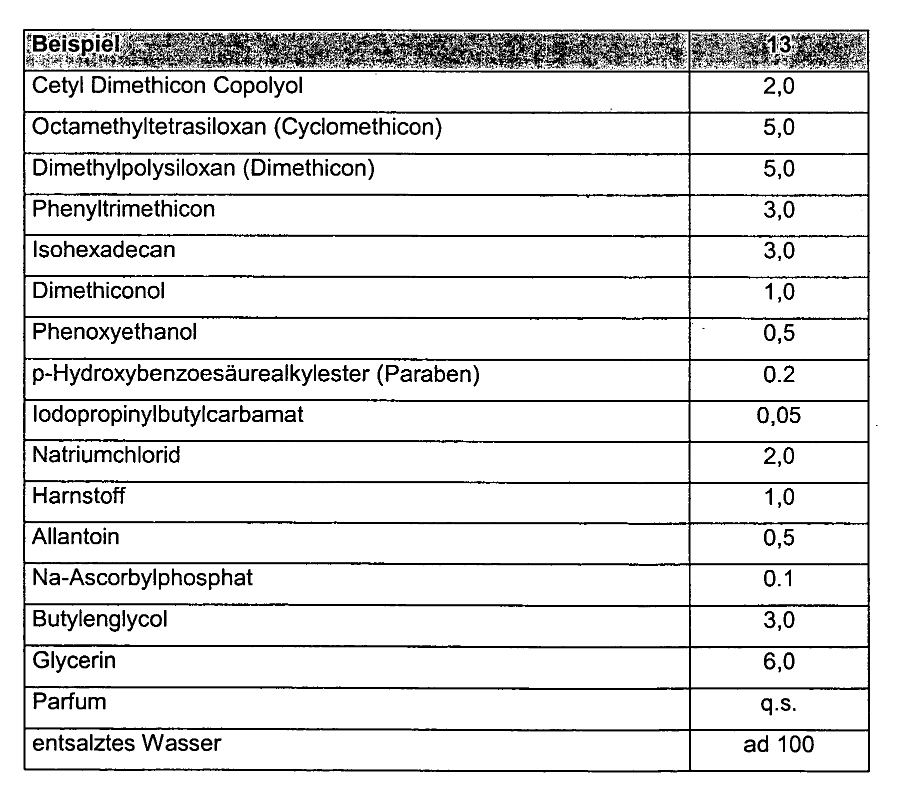

## Patentansprüche

1. Kosmetikum aus
a) einem Spender für pastöse Produkte mit einem eine kosmetische und/oder dermatologische Zubereitung enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, **dadurch gekennzeichnet dass** die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet,
und
b) einer kosmetische Zubereitung enthaltend einen oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen.

2. Kosmetikum nach Anspruch 1, **dadurch gekennzeichnet, dass** in der Zubereitung ein oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen, jeweils in einer Gesamtkonzentration von 0,01 bis 5 Gew.-%, bevorzugt in einer Konzentration von 0,05 bis 3 und ganz besonders bevorzugt in einer Konzentration von 0,1 bis 1 Gew.-%, jeweils bezogen auf das Gesamtgewicht der Zubereitung, enthalten sind.

3. Kosmetikum nach einem der vorhergehenden Ansprüche, **dadurch gekennzeichnet, dass** ein oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen, gewählt werden aus der Gruppe Antioxidantien, Vitamine, Enzyme, Coenzyme, Parfumstoffe, Duftstoffe, Repellentien, Selbstbräuner, ungesättigte Lipide Öle, Fette, Wachse, Polyphenole, Enzyme, Flavonoide und Isoflavonoide, Lignane, Polyole, Polyethylenglykole sowie der Pflanzenextrakte, die einen oder mehrere der vorgenannten Wirkstoffe enthalten

4. Kosmetikum nach einem der vorhergehenden Ansprüche, **dadurch gekennzeichnet, dass** ein oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen, gewählt werden aus der Gruppe bestehend aus Vitamin A und seine Derivate; Vitamin B und seine Derivate; Vitamin C und seine Derivate; Vitamin E und seine Derivate; Vitamin F; Polyphenole, Ubichinon Q 10 (auch in seiner reduzierten Form); 2,6-Ditert.-butyl-4-methylphenol; Dihydroxyaceton; Niacinamid; Pantothensäure und ihre Salze; Panthenol; γ-Oryzanol; Biotin; Kreatin, Kreatinin; Subtilisin; α-Glycosylrutin; Allantoin; Tannin; Azulen; Bisabolol; Glycyrrhizin; Hamamelin; Harnstoff; Genistin; Genistein; Daidzin; Daidzein; Carnitin und seine Derivate und Octadecendicarbonsäure.

5. Kosmetikum nach einem der vorhergehenden Ansprüche, **dadurch gekennzeichnet, dass** die Zubereitung in Form einer Emulsion, einer wässrigen Lösung oder als wasserfreie Formulierung vorliegt.

6. Kosmetikum nach einem der vorhergehenden Ansprüche, **dadurch gekennzeichnet, dass** die Zubereitung eine Viskosität von mindestens 500 mPas aufweist.

7. Kosmetikum nach einem der vorhergehenden Ansprüche, **dadurch gekennzeichnet, dass** die kosmetische Zubereitung eine kontinuierliche wässrige Phase umfasst.

8. Kosmetikum nach Anspruch 7, **dadurch gekennzeichnet, dass** die kontinuierliche Phase eine oder mehrere Hydrokolloide umfasst. besonders den Polyacryl- oder Methacrylsäuren und ihren Derivaten und den Polysacchariden und ihren Derivaten, besonders der Cellulose und ihren Derivaten und den Gummen.

9. Kosmetikum nach Anspruch 1 **dadurch gekennzeichnet, dass** die Förderkanalauslassöffnung (58) des Spenders an der Umfangsfläche des Förderschaftes (50) ausgespart ist und dass das Kopfstück (3) eine die Förderkanalauslassöffnung (58) in der Ausgangstellung (0) der Fördereinrichtung abdeckende Buchse aufweist.

10. Kosmetikum nach Anspruch 1 oder 9, wobei der Spender **dadurch gekennzeichnet ist, dass** die Buchse als eine die Fördereinrichtung längsverschieblich führende Führungsbuchse (31) ausgebildet ist, die wenigstens eine mit der Umfangsfläche des Förderschaftes (50) zusammenwirkende Führungsfläche aufweist.

11. Kosmetikum nach einem der vorherigen Ansprüche, **dadurch gekennzeichnet, dass** an dem Kopfstück (3) des Spenders und der Fördereinrichtung Mitnehmermittel (34,57) vorgesehen sind, durch welche die Fördereinrichtung nach Handbetätigung bei Rückstellung des Kopfstücks (3) in die Ausgangsstellung (0) mitgenommen wird.

12. Kosmetikum nach Anspruch 11, wobei der Spender **dadurch gekennzeichnet** ist, **dadurch gekennzeichnet, dass** an der Buchse (31) eine Mitnehmerschulter (57) ausgebildet ist, die mit einem an dem Förderschaft (50) an geformten Mitnehmerkranz (34) zusammenwirkt.

13. Kosmetikum nach Anspruch 12, **dadurch gekennzeichnet, dass** die Mitnehmerschulter (34) endseitig an der Buchse (31) am Übergang zu dem Ausgabekanal (32) und der Mitnehmerkranz (57) im stirnseitigen Endbereich des Förderschaftes (50) vorgesehen sind.

14. Kosmetikum nach einem der vorherigen Ansprüche, wobei der Spender **dadurch gekennzeichnet ist, dass** der Förderkolben (51) den Förderschaft (50) zur Ausbildung einer ringförmigen Anlagefläche radial überragt und dass die Führungsbuchse (31) eine stirnseitige Druckfläche (33) aufweist, die in der Ausgangsstellung (0) mit axialem Abstand zu der Anlagefläche angeordnet und durch axiales Verschieben des Kopfstückes (3) in Richtung auf den Behälter (1) an die Anlagefläche anlegbar ist.

15. Kosmetikum nach einem der vorherigen Ansprüche, wobei der Spender **dadurch gekennzeichnet ist, dass** die Innenwand der Förderkammer (100) durch eine Innenhülse (13) gebildet ist, welche an der kopfstückseitigen Stirnseite des Behälters (1) an der dem Kopfstück (3) zu gewandten Seite an dem Behälter (1) vorgesehen ist.

16. Kosmetikum nach einem der vorherigen Ansprüche, wobei der Spender durch ein Kopfgegenstück (4) **gekennzeichnet** ist, das einen topfförmig auf die Innenhülse (13) gestülpten Haltezylinder (41) sowie einen konzentrisch zu dem Haltezylinder (41) angeordneten, die Gleiterschiebung des Kopfstückes (3) führenden Führungszylinder (42) aufweist.

17. Kosmetikum nach Anspruch 16, wobei der Spender **dadurch gekennzeichnet ist, dass** das förderkammerseitige Ende des Führungszylinders (42) einen Förderkolbenanschlag für den Förderkolben (51) aufweist.

18. Kosmetikum nach einem Anspruch 16 oder 17, wobei der Spender **dadurch gekennzeichnet ist, dass** der Haltezylinder (41) mit einer bodenseitigen Ringschulter (44) versehen ist, die eine Anlagefläche für eine das Kopfstück in der Ausgangsstellung (0) unter Vorspannung haltende Schraubenfeder ausbildet und auf die Stirnseite des Behälters (1) aufgesetzt ist.

19. Kosmetikum nach einem der Ansprüche 16 bis 18, wobei der Spender **dadurch gekennzeichnet ist, dass** das Kopfgegenstück (4) wenigstens einen Anschlag (46a) zur Begrenzung der axialen Verschiebebewegung des Kopfstücks (3) aufweist und zusammen mit dem Kopfstück (3) als vorgefertigte Spenderkomponente ausgebildet und stirnseitig an dem Behälter (1) befestigt ist.

20. Kosmetikum nach Anspruch 19, wobei der Spender **dadurch gekennzeichnet ist, dass** die Spenderkomponente mit dem Behälter (1) über an dem Kopfgegenstück (4) und der Stirnseite des Behälters (1) ausgebildete Rastmittel (47 ; 17) verrastet ist.

21. Kosmetikum nach einem der vorherigen Ansprüche, wobei der Spender **dadurch gekennzeichnet ist, dass** das Kopfstück (3) derart längsverschieblich ist, dass das Kopfstück (3) mittels Handbetätigung von der Ausgangsstellung (0) zunächst um eine erste axiale Wegstrecke (a) zur Anlage an den Förderkolben bei gleichzeitiger Feilegung der Förderkanalauslassöffnung (58) in dem Ausgabekanal (32) in eine Mittelposition (M) bringbar ist und das Kopfstück (3) danach bei fortschreitender axialer Verschiebung unter Mitnahme des Förderkolbens (51) von der Mittelposition (M) in eine Ausgabe-Endposition (V) bringbar ist, in welcher die Förderkammer (100) durch Verschiebung des Förderkolbens (51) ihr kleinstes Volumen erreicht hat.

22. Kosmetikum nach einem der vorherigen Ansprüche, wobei der Spender durch ein an dem Kopfteil befestigtes Verschließteil (60) **gekennzeichnet ist, durch** welches eine Produktabgabeöffnung (39) des Ausgabekanals (32) verschließbar ist.

23. Kosmetikum nach Anspruch 22, wobei der Spender **dadurch gekennzeichnet ist, dass** die Produktabgabeöffnung (39) ringförmig um einen in dem Ausgabekanal angeordneten Verschlussdorn (32a) ausgebildet ist und dass das Verschließteil (60) eine ringförmig ausgebildete, an den Verschlussdorn dichtend anlegbare Dichtlippe aufweist.

24. Kosmetikum nach Anspruch 22 oder 23, wobei der Spender **dadurch gekennzeichnet ist, dass** das Verschließteil (60) aus einer weichelastischen Kunststoffmasse, vorzugsweise aus einem thermoplastischen Elastomer gebildet ist.

25. Kosmetikum nach einem der Ansprüche 22 oder 24, wobei der Spender **dadurch gekennzeichnet ist, dass** das Verschließteil (60) einstückig mit einem zumindest stirnseitig an der Außenseite des Kopfteiles (3) ausgebildeten Überzug (61) ist.

26. Verwendung eines Kosmetikums nach einem der vorhergehenden Ansprüche zur Reinigung und/oder Pflege der Haut sowie der Hautanhangsgebilde.

27. Verwendung eines Spenders nach einem der Ansprüche 1 und/oder 7 bis 26 zur Aufbewahrung und Darreichung kosmetischer und/oder dermatologischer Zubereitungen enthaltend einen oder mehrere Inhaltsstoffe, welche eine oder mehrere der folgenden Eigenschaften aufweisen: I) oxidationsempfindlich gegenüber Sauerstoff oder Luft, II) hydrolyseempfindlich gegenüber Wasser oder Luftfeuchtigkeit, III) die Geruchsrezeptoren von Menschen und/oder Tieren reizen, IV) eine Temperaturänderung unter Einwirkung von Wasser, Luftfeuchtigkeit und/oder Sauerstoff der Zubereitung bewirken, V) ihre physiologische Wirksamkeit unter Einwirkung von Wasser, Luftfeuchtigkeit und/oder Sauerstoff einbüßen.

28. Verwendung eines Spenders nach einem der Ansprüche 1 und/oder 7 bis 24 zur Aufbewahrung und Darreichung kosmetischer und/oder dermatologischer Zubereitungen nach einem der Ansprüche 2 bis 8.
